# EUROPEAN PATENT APPLICATION

(11) **EP 4 039 276 A1**
(43) Date of publication of application: **10.08.2022**
(21) Application number: 20873304.8
(22) Date of filing: 30.09.2020
(51) Int. Cl.: A61K 45/00, A61K 45/06, A61P 35/00, A61P 37/04, A61P 43/00, A61K 31/196, A61K 31/27, A61K 31/341, A61K 31/351, A61K 31/381, A61K 31/40, A61K 31/4025, A61K 31/404, A61K 31/4406, A61K 31/4409, A61K 31/4525, A61K 31/47, A61K 31/4709, A61K 31/472, A61K 31/495

(54) **INHIBITOR AGAINST EXPRESSION OF IMMUNE CHECKPOINT MOLECULE**

(30) Priority: 30.09.2019 JP 2019181014
(71) Applicant: Renascience Inc., Tokyo 103-0023 (JP)
(72) Inventor: ANDO, Kiyoshi, Isehara-shi, Kanagawa 259-1193 (JP); YAHATA, Takashi, Isehara-shi, Kanagawa 259-1193 (JP); MIYATA, Toshio, Sendai-shi, Miyagi 980-8575 (JP)
(74) Representative: Vossius & Partner Patentanwälte Rechtsanwälte mbB
(86) International application number: PCT/JP2020/037285
(87) International publication number: WO 2021/066062

(57) **Abstract**

The present invention provides a novel use of a compound having a plasminogen activator inhibitor-1 (PAI-1) inhibitory effect. Specifically, a compound having a PAI-1 inhibitory effect is used as an immunostimulator, an immune checkpoint inhibitor, an inhibitor for exacerbation of tumor cells caused by PD-L1, or an enhancer of immunotherapy for tumors, based on the inhibitory effect of the compound on expression induction of immune checkpoint molecules.

## Description

### Technical Field

The present invention relates to a novel use of a compound having an inhibitory effect on the activity of plasminogen activator inhibitor-1 (in the present invention, this may be abbreviated as "PAI-1"). More specifically, the present invention relates to use of a compound having a PAI-1 inhibitory effect as an inhibitor for immune checkpoint molecule expression, an immunostimulator, an immune checkpoint inhibitor, or an inhibitor for exacerbation of tumor cells caused by immune checkpoint molecules, based on the inhibitory effect of the compound on induction of the expression of immune checkpoint molecules.

### Background Art

Our bodies have an immune defense mechanism for protecting us by eliminating foreign substances, such as bacteria and viruses that have invaded the body from outside. The immune defense mechanism also plays an important role in eliminating cancer cells that have formed inside the body. However, as cancer progresses, the immune function is depressed, and immunity often fails against cancer cells. Cancer has so far been treated mainly by surgical treatment, chemotherapy (including cell-killing therapy, molecularly targeted therapy, angiogenesis inhibition therapy, and immunotherapy), and radiotherapy. However, such treatment methods all have advantages and disadvantages. Therefore, in addition to these methods, establishment for further new treatment methods has been in demand especially for advanced cancer patients who have depressed immune function as described above.

Various researchers, including Honjo et al., have recently clarified that cancers skillfully use immunosuppressive factors called "immune checkpoint molecules" (also simply referred to below as "ICMs"), such as PD-1 (programmed cell death protein 1), a ligand thereof, which is PD-L1 (programmed cell death-ligand 1), and CTLA-4 (cytotoxic T-lymphocyte-associated protein 4), so as to proliferate, escaping elimination by the immune system (immune surveillance) by cytotoxic T cells etc. It has currently been experimentally proven that unlocking such an immune escape mechanism reactivates (stimulates) immune cells so that the immune cells become enabled to kill cancer cells again. Immune checkpoint inhibitors (also simply referred to below as "ICIs"), which use this concept, have been clinically applied to the treatment of various types of tumors, including lung cancer, malignant melanoma, and kidney cancer. In particular, ICIs have been effective in treatment; for example, some patients have shown a long-term response of more than 2 to 3 years, which has almost never been observed with conventional chemotherapy.

At present, ICIs, which are clinically applied to the treatment of cancer, are antibody drugs targeting PD-1 and PD-L1 as ICMs. PD-L1 is not only highly expressed in various tumor cells, but also expressed in many cells, including antigen-presenting cells, such as macrophages and dendritic cells. On the other hand, its partner, PD-1, is mainly expressed in immune cells, such as cytotoxic T cells. When PD-L1 expressed in tumor cells etc. binds to PD-1 on cytotoxic T cells, the activity of the T cells will be inhibited. Due to this mechanism, even when cytotoxic T cells recognize a tumor as a foreign substance, the T cells whose cytotoxic function is inhibited allow the tumor to escape the original immune surveillance mechanism (immunity escape), resulting in proliferation and exacerbation of the tumor. The antibody drugs mentioned above (anti-PD-1 antibody and anti-PD-Ll antibody) function to inhibit the immune checkpoint mechanism of PD-1 and PD-L1, which inhibits the immune system. In fact, the effect of inhibiting tumor proliferation has been observed in several percent of cancer patients. However, clinical cases have also been reported in which long-term use resulted in the occurrence of resistance, causing tumor exacerbation again; thus, the development of a novel ICI or immunostimulator is in demand. Additionally, the development of a low-molecular-weight compound as an ICI or immunostimulator to replace antibody drugs that are not suitable for oral administration is also in demand.

Plasminogen activator inhibitor-1 (PAI-1) is a serine protease inhibitor that specifically inhibits tissue plasminogen activator (tPA) and urokinase-type plasminogen activator (uPA). PAI-1 suppresses plasmin production and inhibits fibrin degradation. Based on tertiary structural differences, PAI-1 is present in an active form that shows PA inhibitory activity and in a latent form that shows no PA inhibitory activity. In plasma, PAI-1 is known to be typically present in a concentration of 20 ng/mL, and produced in hepatocytes, megakaryocytes, and lipocytes in addition to vascular endothelial cells, which are the primary PAI-1-producing cells.

The structural characteristics of PAI-1 and its gene have been well confirmed. In particular, the DNA sequences encoding the full-length PAI-1 from humans and animals have been cloned and sequenced. For example, the cDNA sequence of human PAI-1 and its encoding amino acid sequence have been registered under Genbank accession no. X047444. Likewise, the cDNA sequence of mouse PAI-1 and its encoding amino acid sequence have been registered under Genbank accession no. M33960. PAI-1 particularly targeted by the present invention is human PAI-1. As a side note, the amino acid residues from #333 (Ser) to #346 (Lys) of PAI-1 are also known to be associated with the inhibitory effect of PAI-1 on plasminogen-activating factors (Non-patent Literature (NPL) 1).

PAI-1 is an acute-phase protein and is thought to be one of the factors that cause ischemic organ dysfunctions in sepsis and disseminated intravascular coagulation syndrome (DIC) through accelerated production due to various cytokines and growth factors. Further, genetic polymorphism involving single-base substitutions in the PAI-1 gene promoter is known, and it has been revealed that such genetic polymorphism causes an increase in plasma PAI-1 concentration.

It has been widely studied and reported that PAI-1 is deeply associated with and acts on renal diseases, such as diabetic nephropathy, chronic kidney disease (CKD), nephrotic syndrome, postrenal renal failure, and pyelonephritis (NPL 2 to NPL 6). In addition, PAI-1 is considered to be associated with the formation and development of pathological conditions of various diseases, such as various thromboses, cancer, diabetes, ocular diseases such as glaucoma and retinopathy, polycystic ovary syndrome, radiation injuries, alopecia (baldness), splenohepatomegaly, and arteriosclerosis (NPL 7 to NPL 12). Further, there is a report stating that PAI-1 is associated with the control of the diurnal rhythm, which is presumably involved in the formation of vascular endothelial cells and the occurrence of events such as cerebral infarction and myocardial infarction (NPL 13 to NPL 15). For this reason, a compound that inhibits PAI-1 activity is useful as a preventive and treatment agent for various diseases such as thrombosis, cancer, diabetes mellitus, diabetic complications, various kidney diseases, ocular diseases such as glaucoma and retinopathy, polycystic ovary syndrome, alopecia, bone-marrow regeneration, splenomegaly due to extramedullary hematopoiesis, amyloidosis, and arteriosclerosis (NPL 16 and NPL 17). Further, NPL 18 states that a low-molecular-weight PAI-1 inhibitor inhibits differentiation of adipose cells, thereby inhibiting the development of diet-induced obesity. Therefore, a PAI-1 inhibitor is presumably effective also for preventing and treating obesity.

Tissue fibril formation occurs in many tissues and organs such as the lungs, heart, blood vessels, liver, and kidneys. A report has disclosed that the progression of pulmonary fibrosis can be suppressed by the administration of a PA or PAI-1 inhibitor to activate the fibrinolysis system (NPL 19). Therefore, a PAI-1 inhibitor is known to be effective for treating tissue fibrosis, in particular pulmonary fibrosis (NPL 20 and NPL 21). Further, it has been reported that the decomposition of Aβ can be promoted by inhibiting PAI-1; this finding suggests that a PAI-1 inhibitor is potentially useful as a drug for treating Alzheimer's disease (NPL 22).

Furthermore, the Applicant has recently found that PAI-1 inhibitors act on stem cells or a microenvironment called "niche" to activate stem cells (Patent Literature (PTL) 1).

However, the relationship between PD-L1 expressed in tumor cells or in cell populations constituting the tumor-surrounding environment (e.g., tumor-associated macrophages and cancer-associated fibroblasts) and PAI-1 is unknown so far, and the effect of PAI-1 inhibitor on PD-L1 expression has not been even predicted.

### Citation List

### Patent Literature

PTL 1: WO2014/171464

### Non-patent Literature

NPL 1: Sancho et al., Conformational studies on plasminogen activator inhibitor (PAI-1) in active, latent, substrate, and cleaved forms, Biochem. 34: 1064-1069 (1995)
NPL 2: Takashi Oda et al., PAI-1 deficiency attenuates the fibrogenic response to ureteral obstruction. Kidney International, Vol. 30 (2001), pp. 587-596
NPL 3: Shunya Matsuo et al., Multifunctionality of PAI-1 in fibrogenesis: Evidence from obstructive nephropathy in PAI-1-overexpressing mice. Kidney International, Vol. 67 (2005), pp. 2221-2238
NPL 4: Y. Huang et al., Noninhibitory PAI-1 enhances plasmin-mediated matrix degradation both in vitro and in experimental nephritis. Kidney International (2006) 70, 515-522
NPL 5: Allison A. et al., Plasminogen Activator Inhibitor-1 in Chronic Kidney Disease: Evidence and Mechanisms of Action. J Am Soc Nephrol 17: 2999-3012, 2006
NPL 6: Joris J. T. H. Roelofs et al., Plasminogen activator inhibitor-1 regulates neutrophil influx during acute pyelonephritis. Kidney International, Vol. 75 (2009), pp. 52-59
NPL 7: Michelle K. et al., Plasminogen activator inhibitor-1 and tumour growth, invasion, and metastasis. Thromb Haemost 2004; 91: 438-49
NPL 8: Dan J., Belyea D., et al., Plasminogen activator inhibitor-1 in the aqueous humor of patients with and without glaucoma. Arch Ophthalmol. 2005 Feb; 123(2):220-4
NPL 9: Anupam Basu et al., Plasminogen Activator Inhibitor-1 (PAI-1) Facilitates Retinal Angiogenesis in a Model of Oxygen-Induced Retinopathy IOVS, October 2009, Vol. 50, No. 10, 4971-4981
NPL 10: Fabien Milliat et al., Essential Role of Plasminogen Activator Inhibitor Type-1 in Radiation Enteropathy. The American Journal of Pathology, Vol. 172, No. 3, March 2008, 691-701
NPL 11: M. Eren et al., Reactive site-dependent phenotypic alterations in plasminogen activator inhibitor-1 transgenic mice. Journal of Thrombosis and Haemostasis, 2007, 5: 1500-1508
NPL 12: Jessica K. Devin et al., Transgenic overexpression of plasminogen activator inhibitor-1 promotes the development of polycystic ovarian changes in female mice. Journal of Molecular Endocrinology (2007) 39, 9-16
NPL 13: Yuko Suzuki et al., Unique secretory dynamics of tissue plasminogen activator and its modulation by plasminogen activator inhibitor-1 in vascular endothelial cells. Blood, January 2009, Volume 113, Number 2, 470-478
NPL 14: Koji Maemura et al., Circadian Rhythms in the CNS and Peripheral Clock Disorders: Role of the Biological Clock in Cardiovascular Diseases. J Pharmacol Sci 103, 134-138 (2007)
NPL 15: John A. Schoenhard et al., Plasminogen activator inhibitor-1 has a circadian rhythm in blind individuals. Thromb Haemost 2007; 98: 479-481
NPL 16: Egelund R. et al., J. Biol. Chem., 276, 13077-13086, 2001
NPL 17: Douglas E. Vaughan et al., PAI-1 Antagonists: Predictable Indications and Unconventional Applications. Current Drug Targets, 2007, 8, 962-970
NPL 18: David L. Crandall et al., Modulation of Adipose Tissue Development by Pharmacological Inhibition of PAI-1. Arterioscler. Thromb. Vasc. Biol. 2006; 26; 2209-2215
NPL 19: D. T. Eitzman et al., J. Clin. Invest. 97, 232-237, 1996
NPL 20: Noboru Hattori et al., Bleomycin-induced pulmonary fibrosis in fibrinogen-null mice. J. Clin. Invest. 106:1341-1350 (2000)
NPL 21: Hisashi Kosaka et al., Interferon-γ is a therapeutic target molecule for prevention of postoperative adhesion formation. Nature Medicine, Volume 14, No. 4, APRIL 2008, 437-441
NPL 22: Jacobsen J. S. et al., Proc Natl Acad Sci USA, 105(25), 8754-9, 2008 Jun 16

### Summary of Invention

### Technical Problem

An object of the present invention is to provide a novel use of compounds having a PAI-1 inhibitory effect (below, collectively referred to as "the PAI-1 inhibitor"). More specifically, an object is to provide a novel use of the PAI-1 inhibitor for pathological conditions associated with ICMs, such as PD-L1, and exacerbation of such pathological conditions. The use includes, for example, a novel use of the PAI-1 inhibitor, i.e., enhancement of immunotherapy for tumors, separately from the anticancer effect.

### Solution to Problem

Focusing on PD-L1, which acts suppressively against the immune system inside the body, the present inventors conducted extensive study to solve the above problems, and found that overexpression of PAI-1 in, or addition of PAI-1 to, tumor cells of mice increases expression of PD-L1 in the tumor cells, and they confirmed that further addition of a PAI-1 inhibitor results in inhibition of PD-L1 expression induction by PAI-1. It was confirmed that this phenomenon occurs not only for PD-L1 expressed in tumor cells but also for PD-L1 expressed in cells constituting the tumor-surrounding environment (e.g., M2 macrophages, tumor-associated macrophages (TAMs), and cancer-associated fibroblasts (CAFs)), especially in M2 macrophages, which are immunosuppressive cells. In particular, administration of the PAI-1 inhibitor reduces the proportion of immunosuppressive cells (M2 macrophages, TAMs, CAFs, regulatory T cells) in tumors, which suggests that the PAI-1 inhibitor functions to inhibit the immune checkpoint mechanism (immune escape mechanism) so as to lead the inside of the tumor to an environment susceptible to immune attack.

The present inventors further found that the same phenomenon is observed not only in mouse tumor cells but also in various types of human tumor cells (e.g., ovarian clear cell adenocarcinoma cells, acute myelogenous leukemia cells, chronic myelogenous leukemia cells, and immortalized fetal renal cells), and that, in particular, oral administration of the PAI-1 inhibitor inhibits induction of PD-L1 expression by PAI-1. Accordingly, the inventors confirmed that the above phenomenon occurs not only *in vitro* but also *in vivo* in mammals.

It has conventionally been known that high PAI-1 levels in tumor tissue correlate with poor prognosis in breast cancer and lung adenocarcinoma. According to the above findings of the present invention, the expression of PAI-1 in tumor cells or cells constituting the tumor-surrounding environment induces the expression of PD-L1, which consequently activates the immune checkpoint mechanism (immune escape mechanism). This is believed to affect the immunosuppression, exacerbation of tumors (e.g., proliferation, infiltration, metastasis, or recurrence of tumor cells), and poor prognosis in tumor patients. Therefore, a compound having an PAI-1 inhibitory effect (PAI-1 inhibitor) can prevent the above phenomenon (immune escape mechanism), and consequently functions, for example, as a PD-L1 expression inhibitor, an immune checkpoint inhibitor (ICI), an immunostimulator, and/or an agent for inhibiting exacerbation of tumor cells caused by PD-L1. Therefore, the PAI-1 inhibitor is believed to be useful especially in supplementing and enhancing immunotherapy for tumors, separately from the anticancer effect.

The present inventors further found that administration of the PAI-1 inhibitor to mice transplanted with tumor tissue expressing a high level of PD-L1 results in shrinkage (regression) of the transplanted tumor tissue at an early stage while non-administration of the PAI-1 inhibitor results in growth of the transplanted tumor tissue over time. Since such an effect is not observed in immunodeficient mice, the PAI-1 inhibitor is believed to act alone to inhibit exacerbation of the tumor by stimulating the immunity of tumor cells expressing PD-L1. It was also confirmed that the tumor regression effect of the PAI-1 inhibitor is further enhanced by using other ICIs, such as anti-PD-1 antibodies, in combination.

The present invention has been completed as a result of further research based on these findings, and the invention encompasses the following embodiments.

### (I) PD-L1 expression inhibitor, immunostimulator, immune checkpoint inhibitor (ICI), and inhibitor for exacerbation of tumor cells caused by PD-L1

(I-1) A PD-L1 expression inhibitor comprising a compound having a PAI-1 inhibitory effect as an active ingredient.

(I-2) The PD-L1 expression inhibitor according to (I-1), which is used as an ICI for PD-L1.

(I-3) The PD-L1 expression inhibitor according to (I-1), which is used as an immunostimulator.

(1-4) The PD-L1 expression inhibitor according to (I-1), which is used as an inhibitor for exacerbation of tumor cells caused by PD-L1.

(I-5) The PD-L1 expression inhibitor according to any one of (I-1) to (I-4), which is used as an enhancer of immunotherapy for tumors.

(I-6) The PD-L1 expression inhibitor according to any one of (I-1) to (I-5), wherein the compound having a PAI-1 inhibitory effect is at least one member selected from the group consisting of a low-molecular-weight compound having a PAI-1 inhibitory effect, a PAI-1 neutralizing antibody and a fragment thereof, a peptide, an antisense oligonucleotide, siRNA, and an aptamer.

(I-7) The PD-L1 expression inhibitor according to (I-6), wherein the low-molecular-weight compound having a PAI-1 inhibitory effect is a compound represented by Formula (I) below or a pharmaceutically acceptable salt, ester, or solvate thereof: 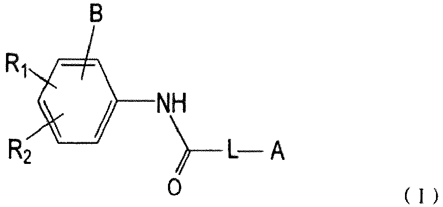 wherein
R₁ and R₂ are the same or different, and each represents hydrogen, halogen, substituted or unsubstituted C₁₋₆ alkyl, C₃₋₈ cycloalkyl, C₃₋₈-cycloalkyl-C₁₋₆-alkyl, C₃₋₈ cycloalkenyl, C₂₋₆ alkynyl, C₃₋₈-cycloalkyl-C₂₋₆-alkynyl, aryl, or 5- to 6-membered ring heteroaryl;
L is a single bond, - [(CH₂)_{M}-O-(CH₂)_{N}]_{Q}-CONH- (wherein M and N are the same or different, and each represents an integer of 1 to 6, and Q represents 0 or 1; -CONH- when Q is 0, and alkyleneoxyalkylene-CONH- when Q is 1), substituted or unsubstituted C₁₋₆ alkylene (some carbon atoms in the alkylene optionally form cycloalkyl), substituted or unsubstituted C₁₋₆ alkylene-O- (some carbon atoms in the alkylene optionally form cycloalkyl), substituted or unsubstituted C₁₋₆ alkylene-NHCO- (in the alkylene-NHCO-, some carbon atoms in the alkylene optionally form cycloalkyl), substituted or unsubstituted C₁₋₆ alkylene-NH-(in the alkylene-NH-, some carbon atoms in the alkylene optionally form cycloalkyl), substituted or unsubstituted C₂₋₆ alkenylene, substituted or unsubstituted C₂₋₆ alkynylene, -CO-, - NH-, 1,4-piperazidinyl, C₁₋₆ alkylene-1,4-piperazidinyl, or adamantylene;

A is a group represented by Formula (I-1) below: 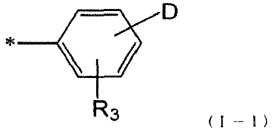 wherein
R₃ are the same or different, and each represents hydrogen, substituted or unsubstituted C₁₋₆ alkyl, or CF₃;
D is substituted or unsubstituted aryl, benzo-fused heteroaryl, or heteroaryl; substituted or unsubstituted C₃₋₈ cycloalkyl or C₃₋₈ heterocycloalkyl; substituted or unsubstituted C₃₋₈ cycloalkenyl or C₃₋₈ heterocycloalkenyl; substituted or unsubstituted C₁₋₆ alkyl; or adamanthyl; and
* is a binding site with L in Formula (I); and
B is COOR₄, wherein R₄ represents hydrogen or a group converted to hydrogen *in vivo.*

(I-8) The PD-L1 expression inhibitor according to (I-7), wherein the compound represented by Formula (I) is a compound of Formulas (I) and (I-1), wherein
B is located at the ortho position of the benzene ring bound to imino,
L is a single bond, and
D is substituted or unsubstituted aryl or benzo-fused heteroaryl.

(I-9) The PD-L1 expression inhibitor according to (I-7) or (1-8), wherein D is phenyl, quinoline, or isoquinolyl.

(I-10) The PD-L1 expression inhibitor according to any one of (I-1) to (I-9), wherein the compound having a PAI-1 inhibitory effect is at least one member selected from the group consisting of 2-[(biphenyl-3-ylcarbonyl)amino]-5-chlorobenzoic acid, 5-chloro-2-({[3-(quinolin-8-yl)phenyl]carbonyl}amino)benzoic acid, and pharmaceutically acceptable salts, esters, and solvates thereof.

(I-11) The PD-L1 expression inhibitor according to any one of (I-1) to (I-10), which is used in combination with at least one member selected from the group consisting of other antitumor agents, other immunostimulators, and other ICIs.

(I-12) The PD-L1 expression inhibitor according to (I-11), wherein the antitumor agents are at least one member selected from the group consisting of antimetabolites, microtubule inhibitors, antitumor antibiotics, topoisomerase inhibitors, platinum-based drugs, alkylating agents, hormone-like drugs, molecular targeted drugs (including antibody drugs and immune checkpoint inhibitors), cytokines, and non-specific immunostimulants.

### (II) Tumor chemotherapy agent

(II-1) A tumor chemotherapy agent comprising at least the PD-L1 expression inhibitor according to any one of (I-1) to (I-12) in combination with at least one member selected from the group consisting of other antitumor agents, other immunostimulators, and other ICIs.

(II-3) The tumor chemotherapy agent according to (II-1), wherein the antitumor agents are at least one member selected from the group consisting of antimetabolites, microtubule inhibitors, antitumor antibiotics, topoisomerase inhibitors, platinum-based drugs, alkylating agents, hormone-like drugs, molecular targeted drugs (including antibody drugs and immune checkpoint inhibitors), cytokines, and non-specific immunostimulants.

### (III) Use of compound having PAI-1 inhibitory effect (#1)

(III-1) Use of a compound having a PAI-1 inhibitory effect for producing a PD-L1 expression inhibitor, an ICI, an immunostimulator, an inhibitor for exacerbation of tumor cells caused by PD-L1, or an enhancer of immunotherapy for tumors.

(III-2) The use according to (III-1), wherein the compound having a PAI-1 inhibitory effect is at least one member selected from the group consisting of a low-molecular-weight compound having a PAI-1 inhibitory effect, a PAI-1 neutralizing antibody and a fragment thereof, a peptide, an antisense oligonucleotide, siRNA, and an aptamer.

(III-3) The use according to (III-2), wherein the low-molecular-weight compound having a PAI-1 inhibitory effect is a compound represented by Formula (I) below or a pharmaceutically acceptable salt, ester, or solvate thereof: 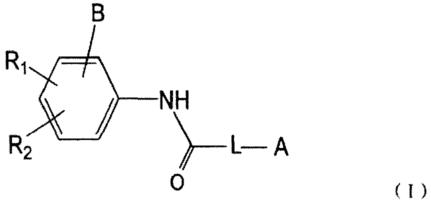 wherein R₁, R₂, L, A, and B are as defined in (I-7) above.

(III-4) The use according to (III-3), wherein the compound represented by Formula (I) is a compound of Formula (I), wherein
B is located at the ortho position of the benzene ring bound to imino, and
L is a single bond.

(III-5) The use according to any one of (III-1) to (III-4), wherein the compound having a PAI-1 inhibitory effect is at least one member selected from the group consisting of 2-[(biphenyl-3-ylcarbonyl)amino]-5-chlorobenzoic acid, 5-chloro-2-({[3-(quinolin-8-yl)phenyl]carbonyl}amino)benzoic acid, and pharmaceutically acceptable salts, esters, and solvates thereof.

(III-6) The use according to any one of (III-1) to (III-5), wherein the compound having a PAI-1 inhibitory effect is used in combination with at least one member selected from the group consisting of other antitumor agents, other immunostimulators, and other ICIs, for producing a PD-L1 expression inhibitor, an ICI, an immunostimulator, or an inhibitor for exacerbation of tumor cells caused by PD-L1.

### (IV) Use of compound having PAI-1 inhibitory effect (#2)

(IV-1) A compound having a PAI-1 inhibitory effect, for use in at least one selected from the group consisting of the following (a) to (e):
(a) inhibiting PD-L1 expression in at least one cell selected from the group consisting of a tumor cell and an immunosuppressive cell;
(b) inhibiting an immune escape mechanism using PD-L1/PD-1 as an immune checkpoint;
(c) stimulating immunity, particularly stimulating immune suppression caused by PD-L1;
(d) inhibiting exacerbation of tumor cells caused by PD-L1; and
(e) enhancing an immunotherapeutic effect on tumors.

(IV-2) The compound for use according to (IV-1), wherein the compound having a PAI-1 inhibitory effect is at least one member selected from the group consisting of a low-molecular-weight compound having a PAI-1 inhibitory effect, a PAI-1 neutralizing antibody and a fragment thereof, a peptide, an antisense oligonucleotide, siRNA, and an aptamer.

(IV-3) The compound for use according to (IV-2), wherein the low-molecular-weight compound having a PAI-1 inhibitory effect is a compound represented by Formula (I) below or a pharmaceutically acceptable salt, ester, or solvate thereof: 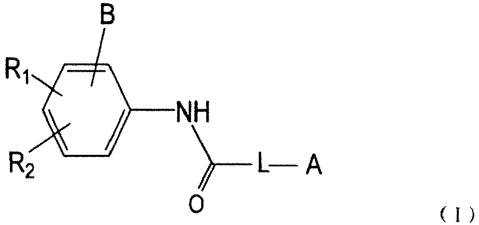 wherein R₁, R₂, L, A, and B are as defined in (1-7) above.

(IV-4) The compound for use according to (IV-3), wherein the compound represented by Formula (I) is a compound of Formula (I), wherein
B is located at the ortho position of the benzene ring bound to imino, and
L is a single bond.

(IV-5) The compound for use according to any one of (IV-1) to (IV-4), wherein the compound having a PAI-1 inhibitory effect is at least one member selected from the group consisting of 2-[(biphenyl-3-ylcarbonyl)amino]-5-chlorobenzoic acid, 5-chlaro-2-({[3-(quinolin-8-yl)phenyl]carbonyl]amino)benzoic acid, and pharmaceutically acceptable salts, esters, and solvates thereof.

(IV-6) The compound for use according to any one of (IV-1) to (IV-5), which is used in combination with at least one member selected from the group consisting of other antitumor agents, other immunostimulators, and other immune checkpoint inhibitors.

### (V) Use of compound having PAI-1 inhibitory effect (#3)

(V-1) A method for inhibiting PD-L1 expression in at least one cell selected from the group consisting of a tumor cell and an immunosuppressive cell in a patient, the method comprising administering an effective amount of a compound having a PAI-1 inhibitory effect to the patient.

(V-2) The method according to (V-1), wherein the immunosuppressive cell is at least one member selected from the group consisting of M2 macrophages, TAM, CAF, and regulatory T cells.

(V-3) The method according to (V-1) or (V-2), wherein the patient is a patient expressing PD-L1 and having at least one cell selected from the group consisting of a tumor cell and an immunosuppressive cell, preferably a human patient.

(V-4) The method according to any one of (V-1) to (V-3), which is a method for inhibiting an immune escape mechanism using PD-L1/PD-1 as an immune checkpoint.

(V-5) The method according to any one of (V-1) to (V-3), which is a method for stimulating suppressed immunity of a tumor patient, particularly stimulating immune suppression caused by PD-L1.

(V-6) The method according to any one of (V-1) to (V-3), which is a method for inhibiting exacerbation of tumor cells caused by PD-L1.

(V-7) The method according to any one of (V-1) to (V-3), which is a method for enhancing an immunotherapeutic effect on tumors.

(V-8) The method according to any one of (V-1) to (V-7), wherein the compound having a PAI-1 inhibitory effect is at least one member selected from the group consisting of a low-molecular-weight compound having a PAI-1 inhibitory effect, a PAI-1 neutralizing antibody and a fragment thereof, a peptide, an antisense oligonucleotide, siRNA, and an aptamer.

(V-9) The method according to (V-7), wherein the low-molecular-weight compound is a compound represented by Formula (I) below or a pharmaceutically acceptable salt, ester, or solvate thereof: 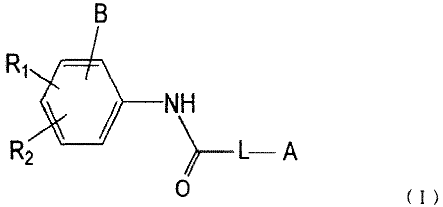 wherein R₁, R₂, L, A, and B are as defined in (I-7) above.

(V-10) The method according to (V-9), wherein the compound represented by Formula (I) is a compound of Formula (I), wherein
B is located at the ortho position of the benzene ring bound to imino, and
L is a single bond.

(V-11) The method according to any one of (V-1) to (V-10), wherein the compound having a PAI-1 inhibitory effect is at least one member selected from the group consisting of 2-[(biphenyl-3-ylcarbonyl)amino]-5-chlorobenzoic acid, 5-chloro-2-({[3-(quinolin-8-yl)phenyl]carbonyl}amino)benzoic acid, and pharmaceutically acceptable salts, esters, and solvates thereof.

(V-12) The method according to any one of (V-1) to (V-11), comprising administering a compound having a PAI-1 inhibitory effect to a patient, simultaneously or in parallel with administration of at least one member selected from the group consisting of other antitumor agents, other immunostimulators, and other ICIs.

### Advantageous Effects of Invention

The present invention is capable of providing a novel use of a compound having a PAI-1 inhibitory effect (PAI-1 inhibitor). Based on its own PAI-1 inhibitory effect, the PAI-1 inhibitor acts to inhibit the induction of PD-L1 expression by PAI-1 in tumor cells and/or immunosuppressive cells, as described below. The PAI-1 inhibitor can provide restoration from the immune system dysfunction (immune depression) caused by PD-L1 by inhibiting the induction of PD-L1 expression by PAI-1. By exerting such an immunostimulatory effect, the PAI-1 inhibitor functions to inhibit and ameliorate exacerbation of the tumor caused by, in particular, PD-L1. Thus, the present invention is capable of providing use of the PAI-1 inhibitor as a PD-L1 expression inhibitor, as an immunostimulator, or as an immune checkpoint inhibitor (ICI) for PD-L1. In addition, the present invention is capable of providing use of the PAI-1 inhibitor as an agent for inhibiting exacerbation of tumor cells caused by PD-L1 (also simply referred to below as "the tumor cell exacerbation inhibitor"), and as an enhancer of immunotherapy for tumors.

Accordingly, the PAI-1 inhibitor can be used alone or in combination with other immunostimulators or ICIs (e.g., anti-PD-1 antibodies) to inhibit the immune escape mechanism of PD-L1. Further, the PAI-1 inhibitor alone or in combination with other antitumor agents, other immunostimulators, or other ICIs is effective in enhancing immunotherapy for tumors and can be used effectively in treating cancer, especially in inhibiting cancer exacerbation (cancer cell proliferation, infiltration, metastasis, and recurrence) or in improving prognosis.

In particular, the PAI-1 inhibitor exerts the above effect by oral administration and is thus useful as an ICI that is used as an alternative to antibody drugs that are unsuitable for oral administration, or as an ICI, an immunostimulator, or an immunotherapy enhancer used in combination with an antibody drug.

### Brief Description of Drawings

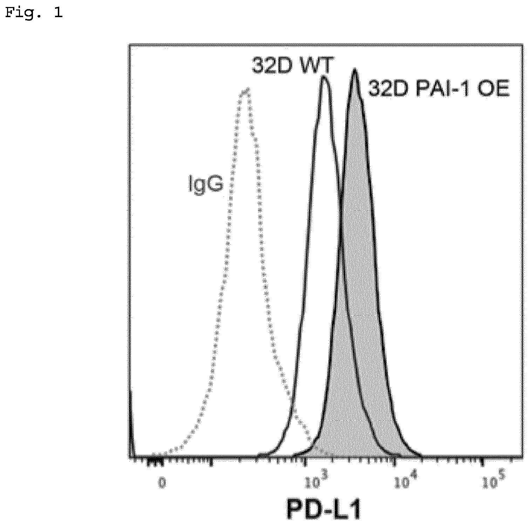

Fig. 1 shows PD-L1 expression levels analyzed by flow cytometry in Experimental Example 1. Fig. 1 shows that PAI-1-overexpressing mouse leukemia cells (32Dp210) (32D PAI-1 OE) had a higher PD-L1 expression level than that of the control, i.e., wild-type cells (32D WT).

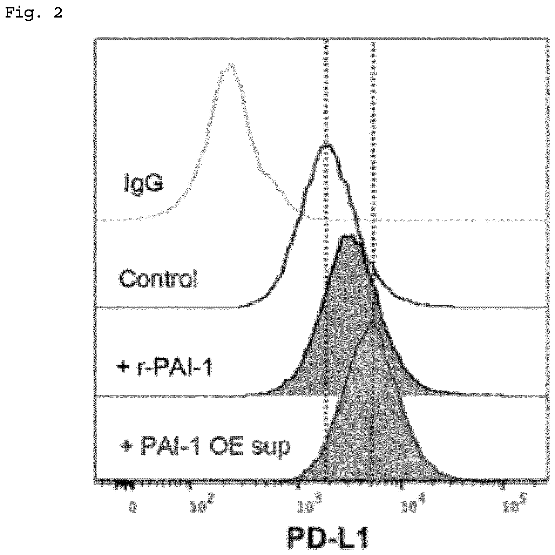

Fig. 2 shows PD-L1 expression levels analyzed by flow cytometry in Experimental Example 2. Fig. 2 shows that 32Dp210 to which PAI-1 was added (+r-PAI-1, +PAI-1 OE sup) both had a higher PD-L1 expression level than that of the same cells to which PAI-1 was not added (untreated) (control).

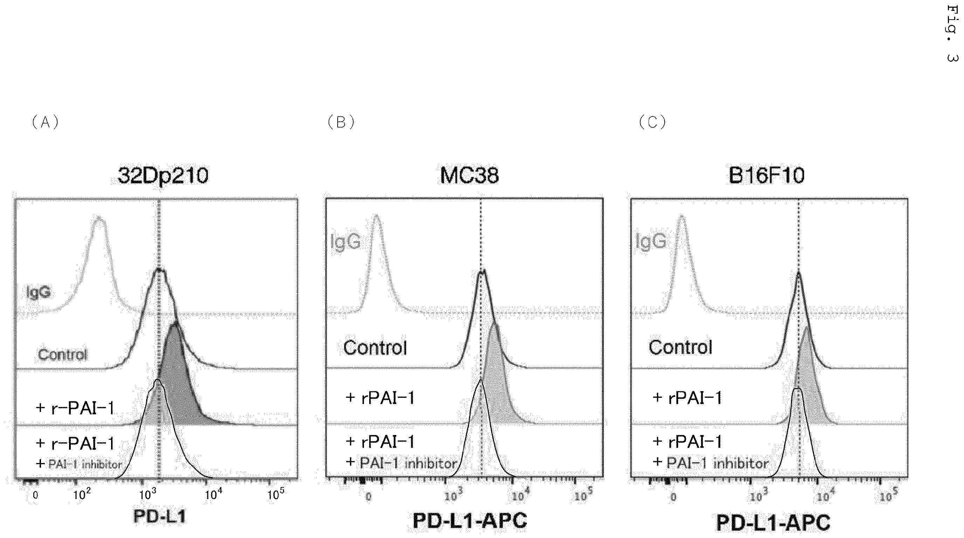

Fig. 3 shows PD-L1 expression levels analyzed by flow cytometry in Experimental Example 3. Fig. 3(A) shows the results of mouse leukemia cells (32Dp210), Fig. 3(B) shows the results of mouse colorectal cancer cells (MC38), and Fig. 3(C) shows the results of mouse malignant melanoma cell line (B16F10). These drawings show that the addition of PAI-1 induced PD-L1 expression in all of the above cells (+r-PAI-1), and that the addition of the PAI-1 inhibitor completely inhibited the induction (+r-PAI-1+PAI-1 inhibitor).

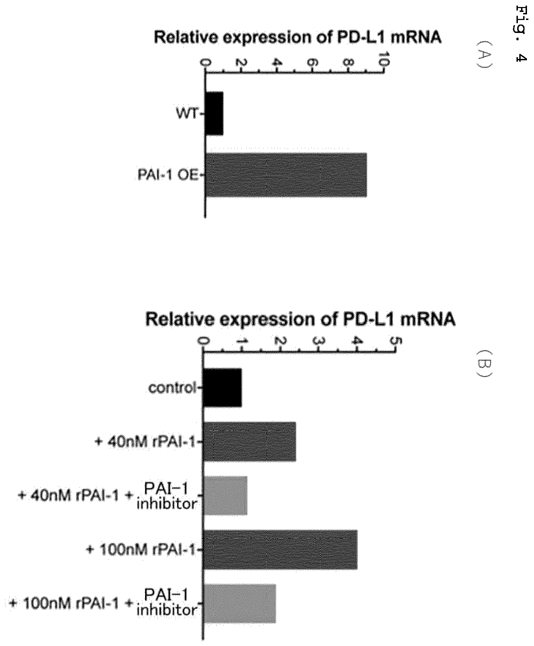

Fig. 4 shows the results of measuring expression levels of PD-L1 mRNA in each cell in Experimental Example 4. Fig. 4(A) shows that 32Dp210 overexpressing PAI-1 (PAI-1 OE) had a higher expression level of PD-L1 mRNA than that of the control, i.e., wild-type cells (WT). Fig. 4(B) shows that expression levels of PD-L1 mRNA in 32Dp210 to which PAI-1 was added (40 nM or 100 nM) (+40 nm rPAI-1, +100 nM rPAI-1) were higher than that of the same cells to which PAI-1 was not added (untreated) (control), and that the addition of the PAI-1 inhibitor inhibited the induction of the expression (+40 nm rPAI-1+PAI-1 inhibitor, +100 nM rPAI-1+PAI-1 inhibitor).

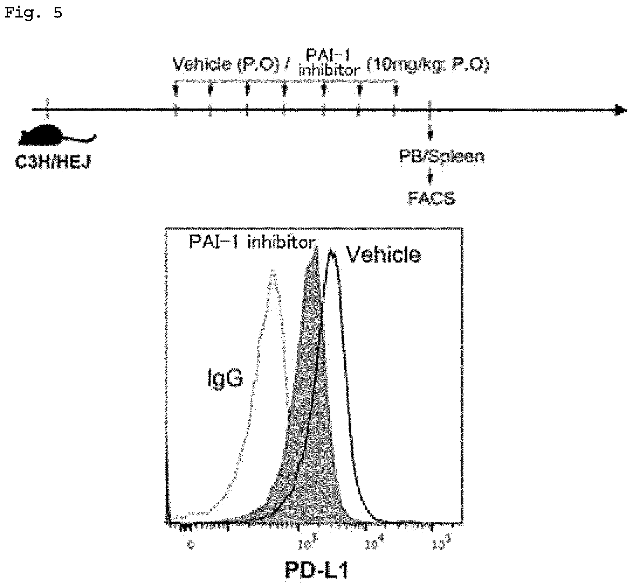

Fig. 5 shows the results of peripheral blood from among PD-L1 expression levels analyzed by flow cytometry in Experimental Example 5. The drawing shows that mice transplanted with mouse leukemia cells (32Dp210) had a reduced PD-L1 expression level when orally administered with the PAI-1 inhibitor, compared with when orally administered with physiological saline instead of the PAI-1 inhibitor (vehicle).

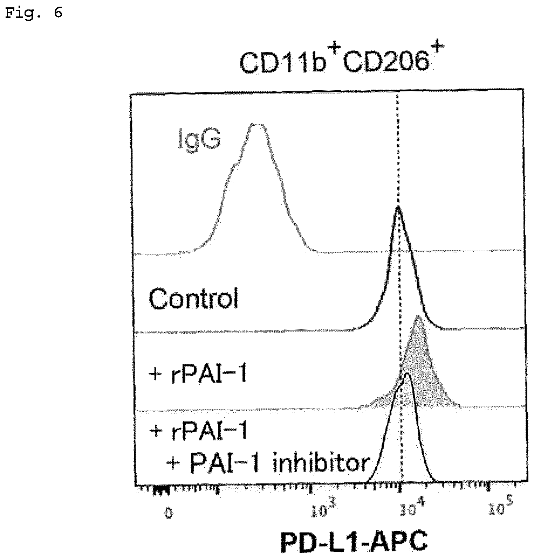

Fig. 6 shows PD-L1 expression levels in M2 macrophages (CD11b⁺CD206⁺) analyzed by flow cytometry in Experimental Example 6. Fig. 6 shows that M2 macrophages to which rPAI-1 was added (+rPAI-1) had an increased PD-L1 expression level, compared with untreated M2 macrophages (control), and that the increase was suppressed by the addition of the PAI-1 inhibitor (+rPAI-1+PAI-1 inhibitor).

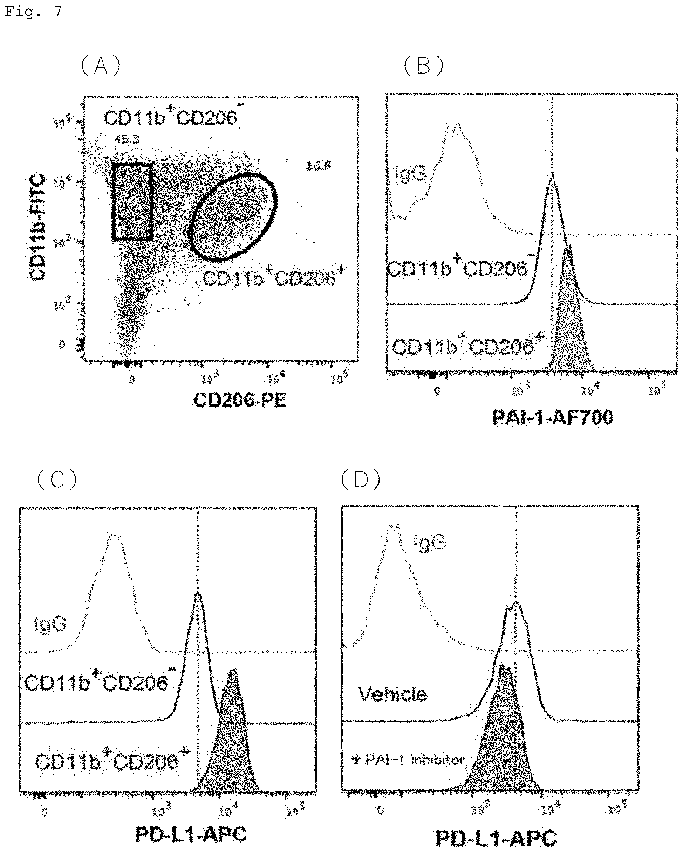

Fig. 7 shows the results of Experimental Example 7. Fig. 7(A) shows the results of gating on M2 macrophages (CD11b⁺CD206⁺) and other macrophages (CD11b⁺CD206⁻) using an anti-CD11b antibody and an anti-CD206 antibody in terms of mouse spleen cells. Fig. 7(B) shows the results of comparing PAI-1 expression levels between M2 macrophages (CD11b⁺CD206⁺) and other macrophages (CD11b⁺CD206⁻). Fig. 7(C) also shows the results of comparing PD-L1 expression levels between those macrophages. Fig. 7(D) shows the results of comparing the PD-L1 expression levels in M2 macrophages (CD11b⁺CD206⁺) in spleen cells in mice to which the PAI-1 inhibitor was orally administered and those to which physiological saline (vehicle) was orally administered.

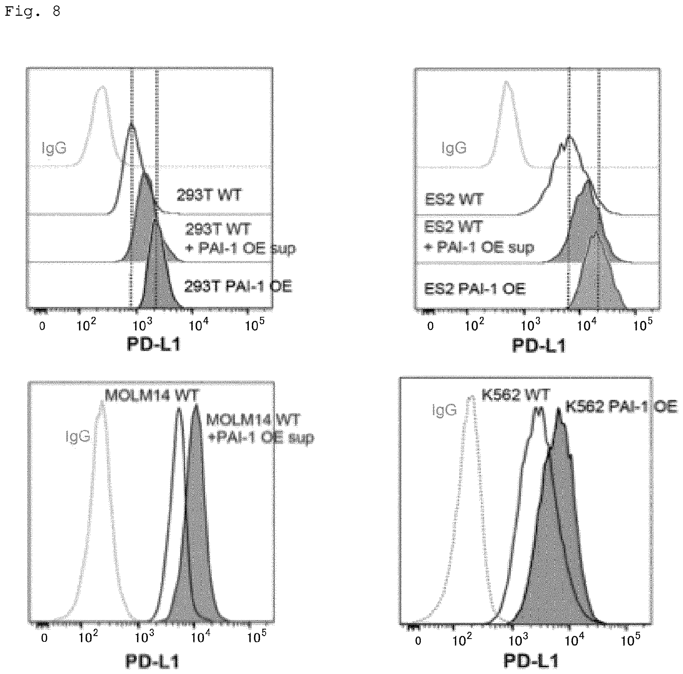

Fig. 8 shows PD-L1 expression levels analyzed by flow cytometry in Experimental Example 8 with respect to various human tumor cells (upper-left graph: 293T (immortalized fetal renal cells); upper-right graph: ES2 (ovarian clear cell adenocarcinoma cells); lower-left graph: MOLM14 (acute myelogenous leukemia cells); and lower-right drawing: K562 (chronic myelogenous leukemia cells)). All of these graphs show that the cells overexpressing PAI-1 (PAI-1 OE) and/or the cells to which PAI-1 was added (+PAI-1 OE sup) had a higher PD-L1 expression level than that of the same cells of wild type (WT).

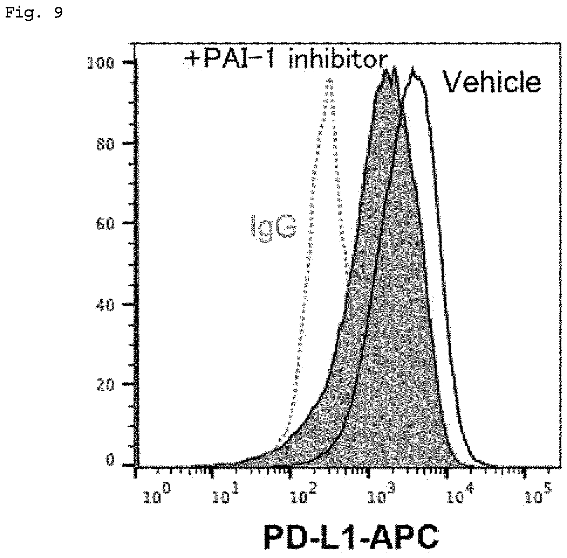

Fig. 9 shows the results of flow cytometry in Experimental Example 9, comparing the PD-L1 expression levels in cancer cells in a tumor mass collected after mice were transplanted with human-derived ovarian cancer cells, engraftment was confirmed, and the PAI-1 inhibitor or physiological saline (vehicle) was orally administered.

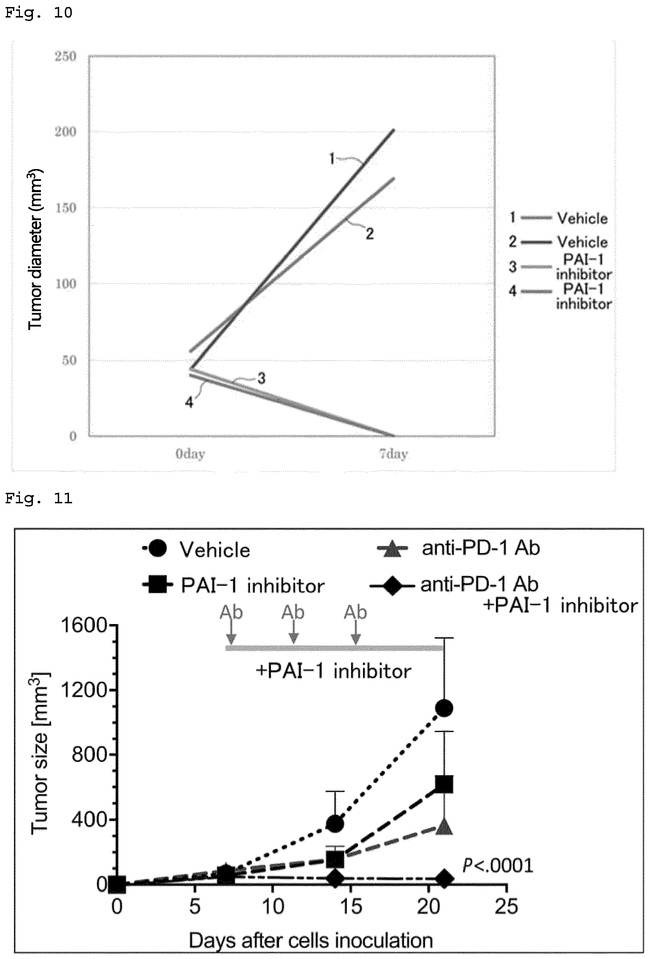

Fig. 10 shows the results of measuring the size of the transplanted tumor mass after the PAI-1 inhibitor or physiological saline (vehicle) was orally administered to mice transplanted with mouse leukemia cells in Experimental Example 10. The drawing shows an increase in the tumor diameter in the mouse groups (n = 2) to which only physiological saline (vehicle) was administered (codes 1 and 2), as well as tumor mass disappearance in the mouse groups (n = 2) to which the PAI-1 inhibitor was administered (codes 3 and 4).
Fig. 11 shows the results of measuring the size of the transplanted tumor mass with respect to mice transplanted with mouse colorectal cancer cells in Experimental Example 11 ((1) an untreated group (vehicle), 2) a single-administration group of anti-PDl antibody (anti-PD-1 Ab), 3) a single-administration group of PAI-1 inhibitor (PAI-1 inhibitor), and 4) a combination group of anti-PDl antibody and PAI-1 inhibitor (anti-PD-1 Ab+PAI-1 inhibitor)). The drawing shows that the tumor mass significantly regressed in the single-administration group of anti-PDl antibody and the single-administration group of PAI-1 inhibitor, and that the tumor mass completely disappeared in the combination group.

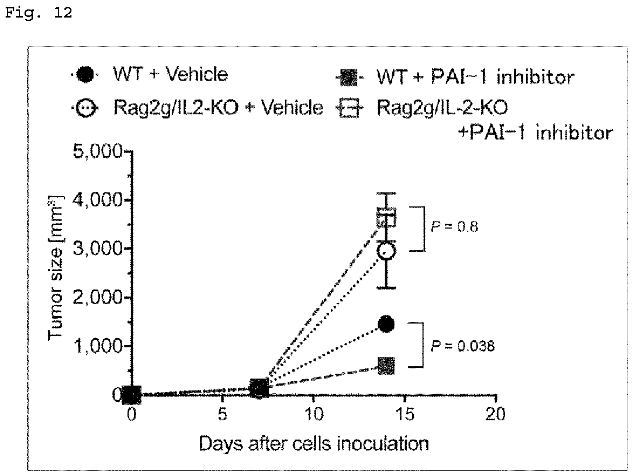

Fig. 12 shows the results of Experimental Example 12. The drawing shows measurement results in terms of how the administration of the PAI-1 inhibitor changed the size of the tumor mass (mouse colorectal cancer cells) subcutaneously transplanted and engrafted into immunodeficient mice (Rag2/IL-2R-KO) and wild-type mice (WT). The drawing shows that administration of the PAI-1 inhibitor achieved a tumor regression effect on wild-type mice, but no such an effect was observed on immunodeficient mice.

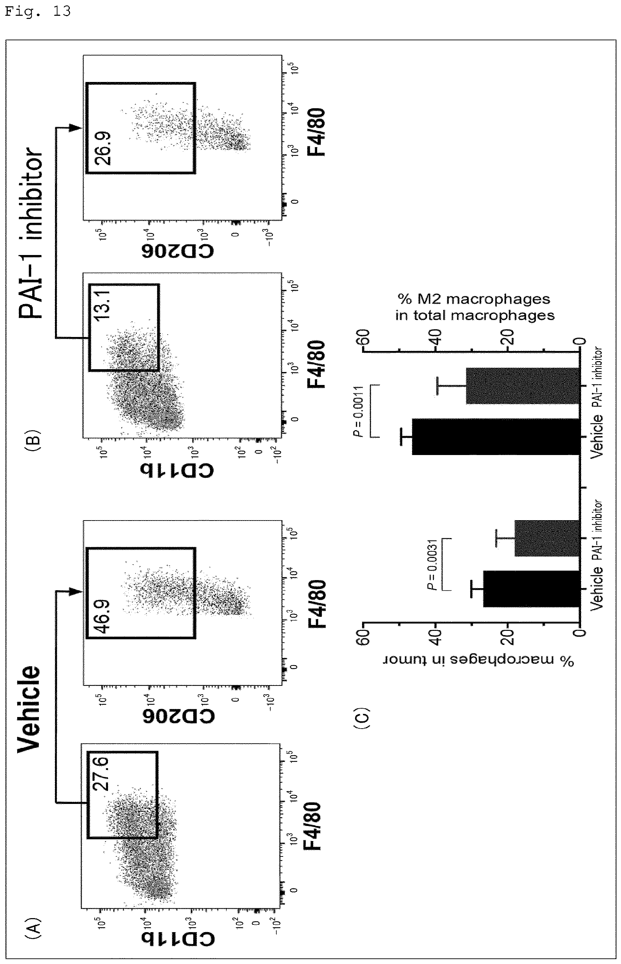

Fig. 13 shows the results of Experimental Example 13. Figs. 13(A) and 13(B) show the percentage of M2 macrophages in the tumor mass analyzed by flow cytometry. The tumors were collected from the cancer-bearing mice of 1) the untreated group and 3) the single-administration group of PAI-1 inhibitor (PAI-1 inhibitor) of Experimental Example 11, and the M2 macrophages were stained with an anti-CDllb antibody, an anti-F4/80 antibody, and an anti-CD206 antibody. Fig. 13(C) is a summary of the results. The bar graph on the left side compares the percentages of macrophages in the tumor between the untreated group (vehicle) and the single-administration group of PAI-1 inhibitor (PAI-1 inhibitor). The bar graph on the right side compares the percentages of M2 macrophages in the total macrophages between the untreated group (vehicle) and the single-administration group of PAI-1 inhibitor (PAI-1 inhibitor).

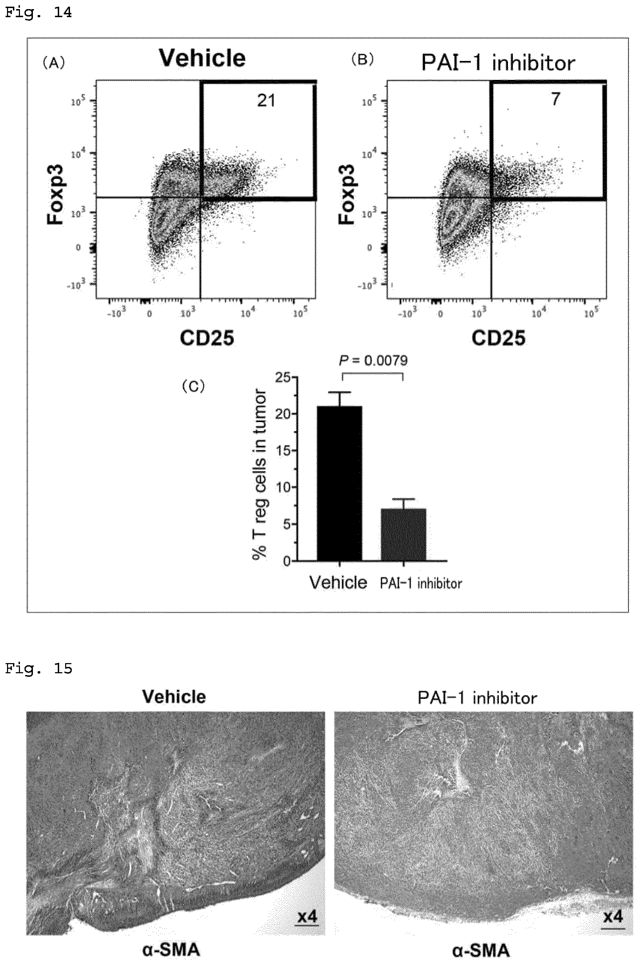

Fig. 14 shows the results of Experimental Example 14. Fig. 14(A) and 14(B) show the percentage of regulatory T cells (T reg cells) in the tumor mass analyzed by flow cytometry. The tumors were collected from the cancer-bearing mice of 1) the untreated group and 3) the single-administration group of PAI-1 inhibitor (PAI-1 inhibitor) of Experimental Example 11, and the cells were stained with an anti-CD25 antibody, an anti-foxp3 antibody, etc. Fig. 14(C) is a summary of the results. Fig. 14(C) is a bar graph comparing the percentages of regulatory T cells in the tumor between the untreated group (vehicle) and the single-administration group of PAI-1 inhibitor (PAI-1 inhibitor).
Fig. 15 shows the results of Experimental Example 15. Fig. 15 shows images of tissue sections prepared from the tumors collected from the cancer-bearing mice of 1) the untreated group (vehicle) and 3) the single-administration group of PAI-1 inhibitor (PAI-1 inhibitor) of Experimental Example 11, stained with an anti-SMAa antibody, and observed under a microscope. The densely stained cells are cancer-associated fibroblasts (CAFs). It is shown that administration of the PAI-1 inhibitor reduced the percentage of intratumoral infiltration of CAFs.

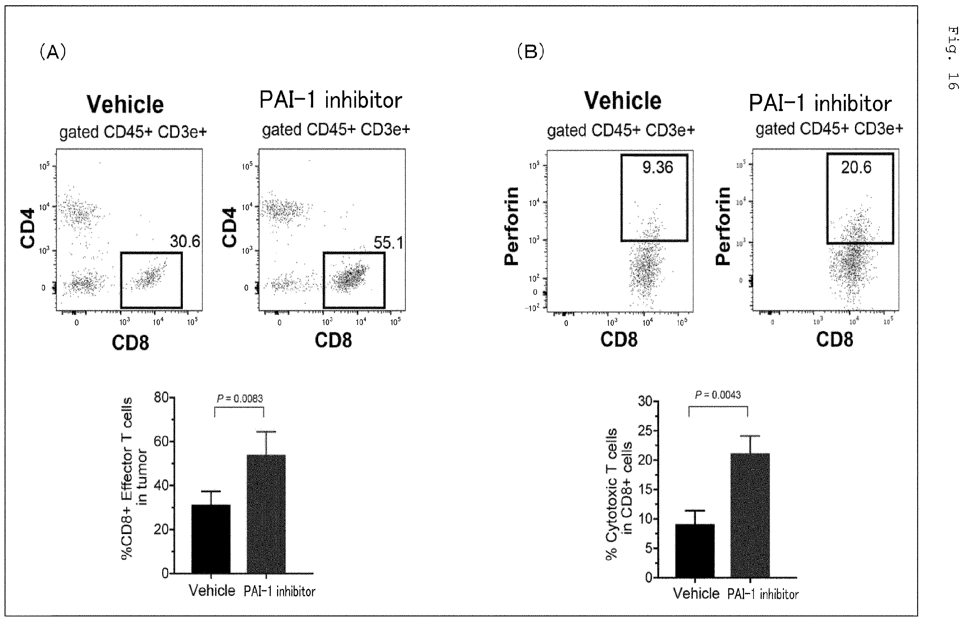

Fig. 16 shows the results of Experimental Example 16. Fig. 16 shows the results of flow cytometry analysis in terms of the percentage of cytotoxic T cells in a tumor mass. The tumors were collected from the cancer-bearing mice of 1) the untreated group (vehicle) and 3) the single-administration group of PAI-1 inhibitor (PAI-1 inhibitor) of Experimental Example 11, and the T cells were stained with an anti-CD45 antibody, an anti-CD3 antibody, an anti-CD4 antibody, an anti-CD8 antibody, and an anti-perforin antibody. Fig. 16(A) shows the results of comparing the percentages (%) of CD8⁺ T cells (CD8⁺ effector T cells) in the tumor between the untreated group (vehicle) and the single-administration group of PAI-1 inhibitor (PAI-1 inhibitor). Fig. 16(B) shows the results of comparing the percentages (%) of cytotoxic T cells in CD8⁺ T cells between the untreated group (vehicle) and the single-administration group of PAI-1 inhibitor (PAI-1 inhibitor).

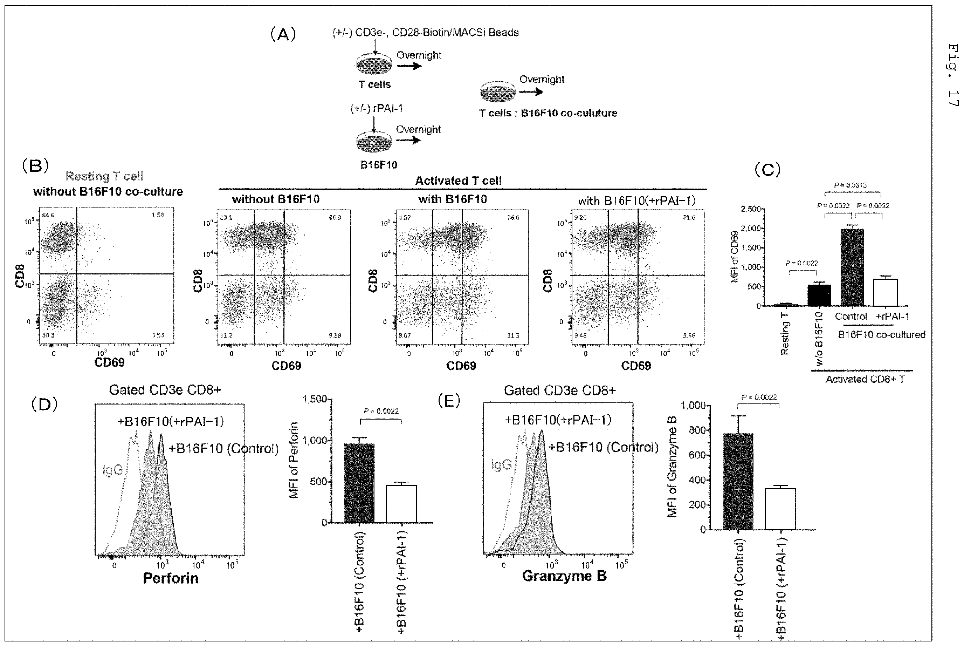

Fig. 17 shows the results of Experimental Example 17. Fig. 17(A) shows a series of procedures for cell preparation. Fig. 17(B) shows the results of flow cytometry analysis obtained by staining the prepared cells (activated T cells, resting T cells) with an anti-CD3 antibody, an anti-CD8 antibody, an anti-CD69 antibody, an anti-perforin antibody, and an anti-granzyme B antibody. The resting T cells are T cells that were not stimulated with antibodies. Fig. 17(C) shows the results of analyzing the degree of T-cell activation using the expression level of CD69 antigen as an index. Fig. 17(D) shows the results of analyzing the expression level of perforin, and Fig. 17(E) shows the results of analyzing the expression level of Granzyme B.

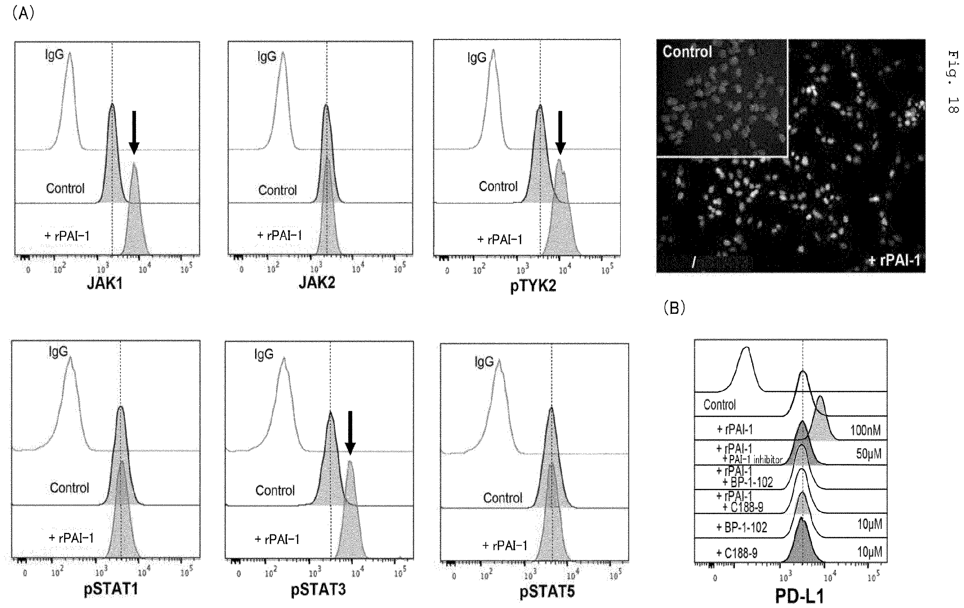

Fig. 18 shows the results of Experimental Example 18. Fig. 18(A) shows the results of measuring the expression levels of JAK1, JAK2, pTYK2, pSTAT1, pSTST3, and pSTAT5 by adding rPAI-1 to a culture medium of cancer cell line (B16F10). The results shown in Fig. 18(B) indicate that the increase in PD-L1 expression induced by rPAI-1 was suppressed by simultaneous addition of rPAI-1 and a STAT3 inhibitor (BP-1-102, C188-9).

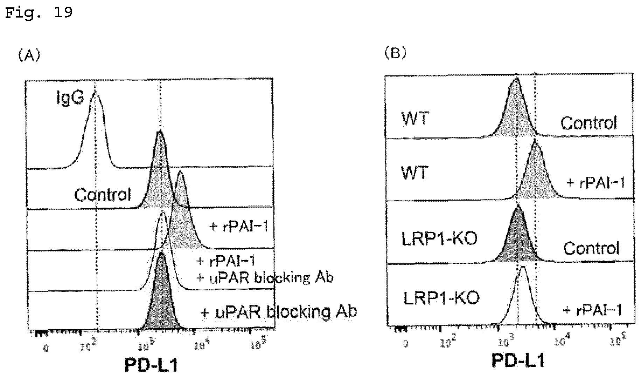

Fig. 19 shows the results of Experimental Example 19. Fig. 19(A) shows that PD-L1 expression induction by rPAI-1 was canceled when a neutralizing antibody that blocks the binding of rPAI-1, uPA, and uPAR (uPAR blocking Ab) was added to a culture medium of cancer cell line (B16F10). Fig. 19(B) shows that PD-L1 expression induction by rPAI-1 of a wild-type cancer cell line (WT) was canceled when rPAI-1 was added to a culture medium of a LRP1-deficient cancer cell line (B16F10; LRP1-KO).

### Description of Embodiments

### (I) Compound Having PAI-1 Inhibitory Effect

The compound having a PAI-1 inhibitory effect targeted by the present invention has the effect of directly or indirectly inhibiting the activity of PAI-1. The term "inhibiting" includes any meaning of inhibiting the activity of PAI-1 itself, and inhibiting the expression of PAI-1 gene and the production of PAI-1 protein. That is, the compound targeted by the present invention may be one that inhibits the activity of PAI-1, regardless of the cause. The term "inhibiting" also includes not only complete elimination of PAI-1 activity and expression, but also partial elimination (suppression, attenuation, or decrease), namely downregulation. In addition, in the present invention, the term "compound" is understood in the broadest sense, and refers to, for example, a substance composed of two or more elements in which the atoms of the elements are bonded and present in a specific ratio. Therefore, examples of the compound include low-molecular-weight chemical compounds, high-molecular-weight chemical compounds, nucleic acids (oligonucleotides), peptides, proteins, antibodies and fragments thereof, and mixtures thereof.

The compound having a PAI-1 inhibitory effect of the present invention includes low-molecular-weight compounds having a PAI-1 inhibitory effect, PAI-1 neutralizing antibodies and fragments thereof, peptides, antisense oligonucleotides, siRNA, and aptamers. These compounds may be compounds that have an effect of inhibiting PAI-1 activity, and include not only compounds conventionally known as PAI-1 inhibitors but also compounds potentially having a PAI-1 inhibitory effect. Hereinafter, these are also referred to collectively as "PAI-1 inhibitor."

The PAI-1 inhibitory activity of the compound to be targeted (the target compound) can be evaluated using an *in vitro* assay system. Examples of the *in vitro* assay system include a method for measuring the change in PAI-1 activity to t-PA in the presence of the target compound. The change in PAI-1 activity can be measured by setting, as an index, a reaction product produced by the action of t-PA on a substrate. Although there is no limitation, for example, Reference Experimental Examples, described later, show an in *vitro* assay system for measuring the change in PAI-1 activity by setting, as an index, the amount of p-nitroaniline (reaction product) produced by the action of t-PA on a coloring substrate (S-2288). The lower the amount of the reaction product, the more strongly t-PA activity is inhibited. Accordingly, in this case, it can be determined that the PAI-1 inhibitory activity of the target compound is higher.

The evaluation of PAI-1 inhibitory activity of the target compound can also be carried out by measuring the change in formation of a complex of PAI-1 and t-PA (PAI-1/t-PA complex) in the presence of the target compound using, for example, western blotting. Here, it can be determined that the smaller the amount of formation of PAI-1/t-PA complex (PAI-1/t-PA complex formation inhibition), the higher the PAI-1 inhibitory activity of the target compound.

Further, whether the target compound inhibits the expression of PAI-1 can be measured by known methods, such as fibrin overlay assay, reverse fibrin overlay assay, or enzyme-linked immunosorbent assay (ELISA).

Among such compounds having PAI-1 inhibitory activity (PAI-1 inhibitors), specific examples of low-molecular-weight compounds include, but are not limited to, (I) the compound represented by Formula (I) in the international publication pamphlet (WO2010/113022) internationally filed on March 31, 2010 (PCT/IB2010/000731) and internationally published on October 7, 2010; (II) the compound represented by Formula (I) in the international publication pamphlet (WO2009/013915) internationally filed on March 12, 2008 (PCT/JP2008/054543) and internationally published on January 29, 2009; and (III) the compound represented by Formula (I) in the international publication pamphlet (WO2009/123241) internationally filed on March 31, 2009 (PCT/JP2009/056755) and internationally published on October 9, 2009. The contents described in these pamphlets are incorporated herein by reference.

In the present specification, the compound represented by Formula (I) in the international publication pamphlet (I), a pharmaceutically acceptable salt or ester thereof, and a solvate thereof are collectively called "the compound group 1." The compound represented by Formula (I) in the international publication pamphlet (II), a pharmaceutically acceptable salt or ester thereof, and a solvate thereof are collectively called "the compound group 2." The compound represented by Formula (I) in the international publication pamphlet (III), a pharmaceutically acceptable salt or ester thereof, and a solvate thereof are collectively called "the compound group 3."

Among these compound groups, examples of compounds belonging to the compound group 1 are shown below.

The compounds belonging to the compound group 1 include a compound represented by Formula (I) below: 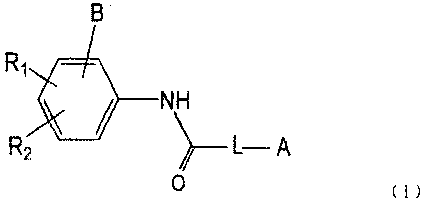 wherein
R₁ and R₂ are the same or different, and each represents hydrogen, halogen, substituted or unsubstituted C₁₋₆ alkyl, C₃₋₈ cycloalkyl, C₃₋₈-cycloalkyl-C₁₋₆-alkyl, C₃₋₈ cycloalkenyl, C₂₋₆ alkynyl, C₃₋₈-cycloalkyl-C₂₋₆-alkynyl, aryl, or 5- to 6-membered ring heteroaryl;
L is a single bond, -[(CH₂)_{M}-O-(CH₂)_{N}]_{Q}-CONH- (wherein M and N are the same or different, and each represents an integer of 1 to 6, and Q represents 0 or 1; -CONH- when Q is 0, and alkyleneoxyalkylene-CONH- when Q is 1), substituted or unsubstituted C₁₋₆ alkylene (some carbon atoms in the alkylene optionally form cycloalkyl), substituted or unsubstituted C₁₋₆ alkylene-O- (some carbon atoms in the alkylene optionally form cycloalkyl), substituted or unsubstituted C₁₋₆ alkylene-NHCO- (in the alkylene-NHCO-, some carbon atoms in the alkylene optionally form cycloalkyl), substituted or unsubstituted C₁₋₆ alkylene-NH- (in the alkylene-NH-, some carbon atoms in the alkylene optionally form cycloalkyl), substituted or unsubstituted C₂₋₆ alkenylene, substituted or unsubstituted C₂₋₆ alkynylene, -CO-, -NH-, 1,4-piperazidinyl, C₁₋₆ alkylene-1,4-piperazidinyl, or adamantylene;
A is a group represented by Formula (I-1) below: 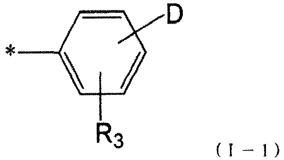 wherein
   R₃ are the same or different, and each represents hydrogen, substituted or unsubstituted C₁₋₆ alkyl, or CF₃;
   D is substituted or unsubstituted aryl, benzo-fused heteroaryl, or heteroaryl; substituted or unsubstituted C₃₋₈ cycloalkyl or C₃₋₈ heterocycloalkyl; substituted or unsubstituted C₃₋₈ cycloalkenyl or C₃₋₈ heterocycloalkenyl; C₁₋₆ alkyl; or adamanthyl; and
   * is a binding site with L in Formula (I); and
   B is COOR₄, wherein R₄ represents hydrogen or a group converted to hydrogen *in vivo.*

The meaning of each group represented by these characters and specific examples thereof are described below.

Examples of the "alkyl" represented by R₁ to R₇ and D, unless otherwise specified, generally include C₁₋₆ linear or branched alkyl groups. Preferable groups are Cₗ-₄ lower alkyl groups such as methyl, ethyl, propyl, isopropyl, butyl, and isobutyl; more preferable are methyl and ethyl; and particularly preferable is methyl.

Any of these alkyls, particularly the "alkyl" represented by R₃ or D, optionally has one or more substituents. Examples of such substituents include halogen, C₁₋₆ alkoxy, halogen-substituted C₁₋₆ alkoxy, hydroxyl, CF₃, CF₃O, CHF₂O CF₃CH₂O, cyano, carboxy, and C₁₋₆ alkoxycarbonyl. The "alkoxy" or the "alkoxy" in the "alkoxycarbonyl" is preferably methoxy, ethoxy, 1-propoxy, or 2-propoxy; and more preferably methoxy.

The "alkyl" represented by R₃ includes C₃₋₆ branched alkyl among the "alkyl" explained above. A preferable example of such branched alkyl is t-butyl.

Examples of the "cycloalkyl" represented by R₁, R₂, R₇, or D or the "cycloalkyl ring" formed by some carbon atoms in the alkylene of L generally include C₃₋₈, and preferably C₃₋₆ cyclic alkyl. Among these, the "cycloalkyl" represented by D and the "cycloalkyl ring" formed by some carbon atoms in the alkylene of L may have one or two substituents at any position. Examples of such substituents include halogen, Cₗ-₄ alkyl, C₁₋₄ halogen-substituted alkyl, Cₗ-₄ alkoxy, C₁₋₄ halogen-substituted alkoxy, hydroxyl, CF₃, CF₃O, CHF₂O, CF₃CH₂O, cyano, carboxy, and alkoxycarbonyl. Here, the meanings of the "alkoxy" and the "alkoxy" in the "alkoxycarbonyl" are as described above.

Examples of the "heterocycloalkyl" represented by D include 3- to 8-membered ring cycloalkyl having one or more identical or different heteroatoms selected from the group consisting of nitrogen, oxygen, and sulfur. 5- to 6-Membered ring cycloalkyl having nitrogen is preferable, and pyrrolidinyl is more preferable.

As is the case with the cycloalkyl described above, the cycloheteroalkyl may have one or two substituents at any position. Examples of such substituents are the same as those for the cycloalkyl.

Examples of the "C₃₋₈-cycloalkyl-C₁₋₆-alkyl" represented by R₁ or R₂ include C₁₋₆ alkyl having C₃₋₈, and preferably C₃₋₆ cycloalkyl as a substituent. The number of carbon atoms in the alkyl is preferably 1 to 4.

Examples of the "cycloalkenyl" represented by R₁, R₂, or D include cycloalkyl having one or more double bonds, in other words, C₃₋₈ cyclic alkenyl having 1 or 2 double bonds. Preferred is C₃₋₆, and preferably C₅ or C₆ cyclic (5- or 6-membered ring) alkenyl. Preferable examples include C₅ or C₆ cyclic alkenyl having one double bond, and more preferably cyclohexenyl.

Examples of the "heterocycloalkenyl" represented by D include groups having one or two carbon atoms of the aforementioned cycloalkenyl replaced with identical or different heteroatoms selected from the group consisting of nitrogen, oxygen, and sulfur. A preferable example is a C₅ or C₆ cyclic alkenyl having one double bond, and a more preferable example is a group in which one of the carbon atoms in cyclohexenyl is replaced with an oxygen atom.

The "cycloalkenyl" and "heterocycloalkenyl" represented by D may have one or two substituents at any position. Examples of such substituents include halogen, Cₗ-₄ alkyl, Cₗ-₄ halogen-substituted alkyl, Cₗ-₄ alkoxy, Cₗ-₄ halogen-substituted alkoxy, hydroxyl, CF₃, CF₃O, CHF₂O, CF₃CH₂O, cyano, carboxy, and alkoxycarbonyl.

Examples of the "alkynyl" represented by R₁ or R₂ include C₂₋₆ linear or branched alkynyl having a triple bond. Ethynyl is preferable.

Examples of the "C₃₋₈-cycloalkyl-C₂₋₆-alkynyl" represented by R₁ or R₂ include C₂₋₆ alkynyl having C₃₋₈, and preferably C₃₋₆ cycloalkyl as a substituent. The number of carbon atoms in the alkynyl is preferably 2 or 3.

Preferable examples of the "aryl" represented by R_{1,} R₂, R₄, or D include C₆₋₁₄ aromatic hydrocarbon. Preferable are phenyl and naphthyl, and more preferable is phenyl. These groups may have one or more substituents at any position. However, when L is substituted or unsubstituted alkylene-NHCO-, the aryl represented by D is preferably aryl other than "unsubstituted phenyl." An example of such aryl is phenyl having one or more substituents.

Examples of the substituents in the aryl represented by R₁, R₂, or D include halogen, C₁₋₆ alkyl (preferably Cₗ-₄ alkyl) , C₁₋₆ cycloalkyl, C₁₋₆ alkoxy (preferably C₁₋₄ alkoxy), C₁₋₆ cycloalkoxy, C₁₋₆ halogen-substituted alkoxy, hydroxyl, CF₃, CF₃O, CHF₂O, CF₃CH₂O, cyano, carboxy, alkoxycarbonyl, benzoyl, and phenyl. Examples of "cycloalkoxy" include C₃₋₈, and preferably C₃₋₆ cyclic alkoxy.

Examples of the "heteroaryl" represented by R₁, R₂, or D include 3- to 6-membered ring, preferably 5- to 6-membered ring, aryl groups having one or more identical or different heteroatoms selected from the group consisting of nitrogen, oxygen, and sulfur.

These groups may have one or two substituents at any position. Examples of substituents of the heteroaryl include halogen, C₁₋₆ alkyl (preferably Cₗ-₄ alkyl) , C₃₋₈ cycloalkyl, C₁₋₆ alkoxy (preferably Cₗ-₄ alkoxy), C₃₋₈ cycloalkoxy, C₁₋₆ halogen-substituted alkoxy, hydroxyl, CF₃, CF₃O, CHF₂O, CF₃CH₂O, cyano, carboxy, alkoxycarbonyl, benzoyl, phenyl, and phosphonooxymethyl. When the heteroaryl is pyrazolyl or pyrrolyl, the phosphonooxymethyl is a substituent of the "heteroaryl" at the 1-position and is removed *in vivo* and converted to pyrazolyl or pyrrolyl unsubstituted at the 1-position, allowing the pyrazolyl or pyrrolyl to show PAI-1 inhibition activity. In other words, the phosphonooxymethyl is a substituent that serves as a so-called prodrug.

When the substituent of D is cycloalkyl or cycloalkoxy, the substituent may also have a substituent. Examples of such substituents include halogen, C₁₋₆ alkyl, C₁₋₆ alkoxy, C₁₋₆ halogen-substituted alkoxy, hydroxyl, CF₃, CF₃O, CHF₂O, CF₃CH₂O, cyano, carboxy, alkoxycarbonyl, benzoyl, and phenyl.

Examples of the "benzo-fused heteroaryl" represented by D include groups in which the benzene ring is fused with the above-mentioned heteroaryl. The above benzo-fused heteroaryl may have one to three substituents at any position. Examples of such substituents include halogen, Cₗ-₄ alkyl, Cₗ-₄ halogenated alkyl, C₁₋₄ alkoxy, C₁₋₄ halogenated alkoxy, hydroxyl, CF₃, CF₃O, CHF₂O, CF₃CH₂O, aryl (preferably phenyl), halogenated aryl, cyano, carboxy, alkoxycarbonyl having Cₗ-₄ alkoxy, etc.

Examples of the "alkylene" represented by L and the "alkylene" in "alkylene-O-," "alkylene-NH-," "alkylene-NHCO-," "alkyleneoxyalkylene-NHCO-," and "alkylene-piperazidinyl" generally include C₁₋₆, and particularly preferably C₁₋₄, linear or branched alkylene. Methylene and ethylene are preferable. The "alkylene" and the "alkylene" in "alkylene-O-," "alkylene-NH-," "alkylene-NHCO-," and "alkyleneoxyalkylene-NHCO-" may be those in which some of the carbon atoms in the alkylene bind to form a C₃₋₈ cycloalkyl ring (cycloalkane).

Examples of the "alkenylene" represented by L include C₂₋₆ linear or branched alkenylene having 1 to 3 double bonds. Preferable is vinylene.

Examples of the "alkynylene" represented by L include C₂₋₆ linear or branched alkynylene having one triple bond.

The "alkylene," "alkylene-O-," "alkylene-NH-," "alkylene-NHCO-," "alkyleneoxyalkylene-NHCO-," "alkenylene," and "alkynylene" each may have one or two substituents. Examples of such substituents include halogen, C₁₋₄ alkoxy, Cₗ-₄ halogen-substituted alkoxy, hydroxyl, CF₃, CF₃O, CHF₂O, CF₃CH₂O, cyano, carboxy, alkoxycarbonyl, amino, acylamino, benzyloxycarbonylamino (Cbz-NH-), alkoxycarbonylamino (e.g., t-butoxycarbonylamino (tBoc-NH-), methoxycarbonylamino, ethoxycarbonylamino, propoxycarbonylamino, isopropoxycarbonylamino, and butoxypropoxycarbonylamino), acyl, etc.

Examples of the "halogen" represented by R₁ or R₂ include fluorine, chlorine, bromine, and iodine. Preferable are chlorine, bromine, and fluorine; chlorine is more preferable.

Examples of the group represented by B in Formula (I) include, in addition to carboxyl (COOH), (1) alkoxycarbonyl, aryloxycarbonyl, and aralkyloxycarbonyl, which can be converted to carboxyl when absorbed *in vivo;* (2) groups that can be easily converted to carboxyl when absorbed *in vivo;* and (3) groups that have been recognized as a group that is biologically equivalent to carboxyl.

Here, examples of the alkoxycarbonyl, aryloxycarbonyl, and aralkyloxycarbonyl in (1) above include groups that are each represented by COOR₄, wherein R₄ is C₁₋₆ alkyl, aryl (preferably phenyl), or aralkyl (preferably benzyl).

Specific examples of the groups in (2) above include groups represented by COOR₄, wherein R₄ is (5-alkyl-2-oxo-1,3-dioxolen-4-yl)methyl represented by the following formula: 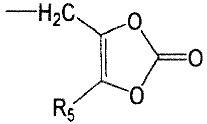 wherein R₅ is C₁₋₆ alkyl;
and a group represented by -CH(R₆)-O-COR₇ or -CH(R₆)-O-CO-OR₇, wherein R₆ is hydrogen or C₁₋₆ alkyl, and R₇ is C₁₋₆ alkyl or C₃₋₈ cycloalkyl.

Examples of the groups in (3) above include heterocyclic groups such as 1H-tetrazol-5-yl, 4,5-dihydro-5-oxo-4H-1,2,4-oxadiazol-3-yl, 4,5-dihydro-5-oxo-1,2,4-thiadiazol-3-yl, and 4,5-dihydro-5-thioxo-4H-1,2,4-oxadiazol-3-yl represented by the following formulas in order from the left (see, for example, Kohara et al. J. Med. Chem., 1996, 39, 5228-5235).

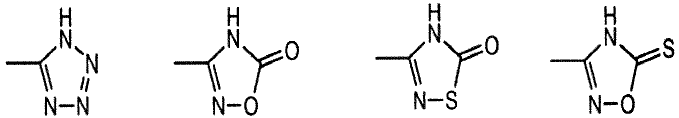

In the present invention, the groups represented by the above formulas are collectively called a "group that is biologically equivalent to carboxyl." In the present specification, salts of the compound represented by Formula (I) and the compound having the above group (group that is biologically equivalent to carboxyl) may be collectively called a bioisostere of the carboxylic acid.

Specific examples of the "alkoxycarbonyl" represented by B (when B represents -COOR₄, wherein R₄ is alkyl) in Formula (I) include t-butoxycarbonyl, methoxycarbonyl, ethoxycarbonyl, propoxycarbonyl, isopropoxycarbonyl, butoxycarbonyl, etc.

The compounds (I) represented by the above formula can be further classified into (I-a) to (I-e) described below depending on the types of L.
(I-a) Compounds wherein L is a single bond;
(I-b) Compounds wherein L is -NH-, -CO-, -CONH-, alkyleneoxyalkylene-CONH-, alkylene-NH-, or alkylene-NHCO-;
(I-c) Compounds wherein L is alkylene-O-;
(I-d) Compounds wherein L is alkylene, alkenylene, or alkynylene; and
(I-e) Compounds wherein L is adamantylene, 1,4-piperazidinyl, or alkylene-1,4-piperazidinyl.

### (I-a) Compounds Wherein L Is Single Bond (Benzenecarboxylic Acid or Bioisosteres Thereof)

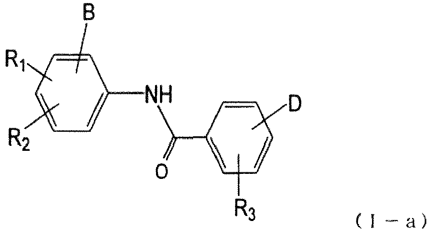

wherein R₁ to R₃, B, and D are as defined above.

In Formula (I-a), B, R₁, and R₂ may be located at any of the ortho, meta, and para positions of the benzene ring to which imino is bound. Preferably B is located at the ortho position of the benzene ring, and R₂ and R₁ are located at the meta and para positions, respectively. When X is sulfur, it is preferable that B is located at the 3-position of the thiophene ring, and that R₂ and R₁ are located at the 4- and 5-positions, respectively.

In Formula (I-a) , R₁ and R₂ are as defined above, preferably are the same or different, and each represents hydrogen, halogen, C₃₋₈-cycloalkyl, C₃₋₈-cycloalkyl-C₁₋₆-alkyl, C₃₋₈-cycloalkyl-C₂₋₆-alkynyl, C₃₋₈-cycloalkenyl, or C₂₋₆-alkynyl. More preferably, R₁ located at the meta position is hydrogen, and R₂ located at the para position is halogen, C₃₋₈-cycloalkyl, C₃₋₈-cycloalkyl-C₁₋₆alkyl, C₃₋₈-cycloalkyl-C₂₋₆-alkynyl, C₃₋₈-cycloalkenyl, or C₂₋₆-alkynyl. R₂ at the para position is preferably halogen.

Here, the halogen is preferably chlorine or bromine, and more preferably chlorine. The C₃₋₈ cycloalkyl is preferably cyclohexyl. The C₃₋₈-cycloalkyl-C₁₋₆-alkyl is preferably C₁₋₆ alkyl having cyclohexyl as a substituent, and more preferably C₁₋₄ alkyl having cyclohexyl as a substituent. The C₃₋₈-cycloalkyl-C₂₋₆-alkynyl is preferably C₂₋₆ alkynyl having cyclohexyl as a substituent, and more preferably C₂₋₃ alkynyl having cyclohexyl as a substituent. The C₃₋₈-cycloalkenyl is preferably a cyclohexenyl, and more preferably cyclohex-1-en-1-yl or cyclohex-6-en-1-yl. The C₂₋₆-alkynyl is preferably C₂₋₄ alkynyl, and more preferably C₂₋₃ alkynyl.

R₃ is as defined above, and is preferably hydrogen.

D is as defined above, and preferable examples thereof include aryl optionally having one or two substituents, benzo-fused heteroaryl optionally having one or two substituents, heteroaryl optionally having one or two substituents, substituted or unsubstituted C₃₋₈ cycloalkyl, C₃₋₈ heterocycloalkyl optionally having one or two substituents, C₃₋₈ cycloalkenyl optionally having one or two substituents, C₃₋₈ heterocycloalkenyl optionally having one or two substituents, and adamanthyl.

Preferable examples of aryl include phenyl optionally having one substituent and naphthyl, and more preferably phenyl. Examples of the substituents are as described above, and preferable are alkyl and alkoxy.

Preferable examples of benzo-fused heteroaryl include quinolyl optionally having one substituent and isoquinolyl. Examples of the substituents are as described above, and preferable are unsubstituted quinolyl and isoquinolyl. There is no limitation to the binding site of quinolyl and isoquinolyl, and in the case of quinolyl, preferable positions are, for example, the 2-position (quinolin-2-yl), 3-position (quinolin-3-yl), 6-position (quinolin-6-yl), and 8-position (quinolin-8-yl); in the case of isoquinolyl, for example, the 4-position (isoquinolin-4-yl) and 5-position (isoquinolin-5-yl) are preferable.

Preferable examples of heteroaryl include pyridyl, thienyl, and furyl, which optionally have one substituent. Examples of the substituents are as described above, and preferable are unsubstituted pyridyl, thienyl, and furyl. Specific examples of pyridyl include pyridin-2-yl, pyridin-3-yl, and pyridin-4-yl; pyridin-4-yl is preferable. Specific examples of thienyl include thiophen-2-yl and thiophen-3-yl; thiophen-2-yl is preferable. Specific examples of furyl include furan-2-yl and furan-3-yl; furan-3-yl is preferable.

Preferable examples of C₃₋₈ cycloalkyl include cyclohexyl optionally having one substituent; and examples of C₃₋₈ heterocycloalkyl include a 5-membered ring having nitrogen as a heteroatom, and preferably pyrrolidinyl optionally having one substituent. Specific examples of pyrrolidinyl include pyrrolidin-1-yl, pyrrolidin-2-yl, pyrrolidin-3-yl, pyrrolidin-4-yl, pyrrolidin-5-yl, and pyrrolidin-6-yl; pyrrolidin-1-yl is preferable. Examples of the substituents are as described above, and preferable are unsubstituted cycloalkyl and heterocycloalkyl.

Preferable examples of C₃₋₈ cycloalkenyl include cyclohexanyl optionally having one substituent. Specific examples of cyclohexanyl include cyclohex-1-en-1-yl, cyclohex-2-en-1-yl, cyclohex-3-en-1-yl, cyclohex-4-en-1-yl, cyclohex-5-en-1-yl, and cyclohex-6-en-1-yl; cyclohex-1-en-1-yl is preferable.

Examples of C₃₋₈ heterocycloalkenyl include a 6-membered ring heterocyclohexanyl with oxygen as a heteroatom optionally having one substituent. Examples of such a group include dihydro-2H-pyranyl, and preferably 3,6-dihydro-2H-pyran-4-yl. Examples of the substituents are as described above, and preferable are unsubstituted cycloalkenyl and heterocycloalkenyl.

Preferable examples of adamanthyl include adamanthyl optionally having one substituent, and the adamanthyl is preferably adamantan-1-yl.

Specific examples of the aromatic carboxylic acid (benzenecarboxylic acid) of the present invention represented by Formula (I-a) or bioisosteres of the carboxylic acid include the following compounds shown in Tables 1 to 6 provided later. Example 2, Example 4, Example 6, Example 7, Example 8, and desalted products thereof, Example 32, Example 33, Example 34, Example 40, Example 42, Example 43, Example 52, Example 56, Example 58, Example 62, Example 63, and desalted products thereof, Example 64 and desalted products thereof, Example 65 and desalted products thereof, Example 66 and desalted products thereof, Example 67 and desalted products thereof, Example 68 and desalted products thereof, Example 69, Example 79, Example 80, Example 81, Example 82, Example 83, Example 87, Example 88, Example 92, Example 93, Example 95, Example 96, Example 97, Example 98, Example 99, and desalted products thereof, Example 100 and desalted products thereof, and Example 102.

### (I-b) Compound Wherein L Is -NH-, -CO-, -CONH-, Alkyleneoxyalkylene-CONH-, Alkylene-NH-, or Alkylene-NHCO-

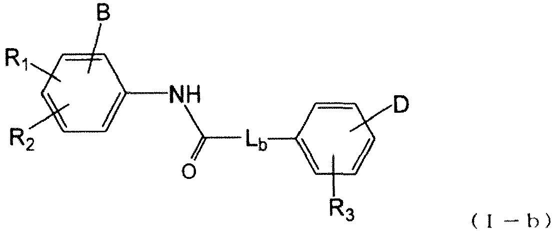

wherein R₁ to R₃, B, and D are as defined above, and L_{b} is a group represented by -NH-, -CO-, -CONH-, substituted or unsubstituted C₁₋₆-alkyleneoxy-C₁₋₆-alkylene-CONH-, C₁₋₆-alkylene-NH-, or substituted or unsubstituted C₁₋₆-alkylene-NHCO-.

Examples of the alkylene represented by "C₁₋₆-alkyleneoxy-C₁₋₆-alkylene-CONH-," "C₁₋₆-alkylene-NH-," and "C₁₋₆-alkylene-NHCO-" include C₁₋₆ alkylene, and preferably C₁₋₄ alkylene. The alkylene may be linear or branched, and some carbon atoms in the alkylene optionally form a C₃₋₈ cycloalkyl ring. The cycloalkyl ring (cycloalkane) is preferably cyclopropane. Linear alkylene is preferable.

The "C₁₋₆-alkyleneoxy-C₁₋₆-alkylene-CONH-," "C₁₋₆-alkylene-NH-," and "C₁₋₆-alkylene-NHCO-" may have one or two substituents in alkylene. The substituents are as defined above, and preferably unsubstituted alkylene.

In Formula (I-b), B, R₁, and R₂ may be located at any of the ortho, meta, and para positions of the benzene ring to which imino is bound. Preferable compounds are those in which B is located at the ortho position of the benzene ring, and R₁ and R₂ are located at the meta and para positions, respectively. R₁ and R₂ are as defined above, preferably are the same or different, and each represents hydrogen or halogen. More preferably, R₁ located at the meta position is hydrogen, and R₂ located at the para position is halogen. The halogen is preferably chlorine, bromine, or fluorine, and more preferably chlorine.

In the compound (I-b) , R₃ is as defined above, and is preferably hydrogen.

In the compound (I-b), D is as defined above, and preferable examples thereof include aryl optionally having one or two substituents, heteroaryl optionally having one or two substituents, and substituted or unsubstituted adamanthyl.

The aryl is preferably phenyl. The substituents are as defined above, and preferably halogen, C₁₋₆ (preferably C₁₋₄) alkyl, or C₁₋₆ (preferably C₁₋₄) alkoxy. Preferable examples of the aryl include unsubstituted phenyl and phenyl having halogen as a substituent. The halogen is preferably chlorine or fluorine, and more preferably fluorine.

The heteroaryl is preferably furyl, and more preferably furan-2-yl or furan-3-yl. Preferable examples of the adamanthyl include adamantan-1-yl. Examples of the substituents of the heteroaryl and adamanthyl are as described above, and unsubstituted heteroaryl and adamanthyl are preferable.

Specific examples of the aromatic carboxylic acid (benzenecarboxylic acid) of the present invention represented by Formula (I-b) or bioisosteres thereof include the following compounds shown in Tables 1 to 6.
Example 71, Example 72, Example 14, Example 106, and desalted products thereof, Example 1, Example 9, Example 10, Example 11, and desalted products thereof, and Example 12.

### (I-c) Compound Wherein L Is Alkylene-O-

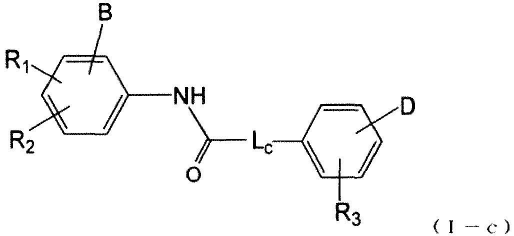

wherein R₁ to R₃, B, and D are as defined above, and L_{c} is a group represented by substituted or unsubstituted C₁₋₆-alkylene-O-.

In the compound (I-c), Lc is C₁₋₆ alkylene-O-, preferably C₁₋₄ alkylene-O-, and more preferably C₁₋₃ alkylene-O-. The alkylene may be linear or branched. The alkylene may have one or two substituents, and is preferably unsubstituted alkylene.

In Formula (I-c), B, R₁, and R₂ may be located at any of the ortho, meta, and para positions of the benzene ring to which imino is bound. Preferable compounds are those in which B is located at the ortho position of the benzene ring, and R₁ and R₂ are located at the meta and para positions, respectively. R₁ and R₂ are as defined above, preferably are the same or different, and each represents hydrogen, halogen, aryl optionally having one or two substituents, or 5- to 6-membered ring heteroaryl optionally having one or two substituents. More preferably, R₁ located at the meta position is hydrogen, and R₂ located at the para position is halogen, aryl optionally having one substituent, or 5- to 6- membered ring heteroaryl optionally having one substituent. R₂ is preferably halogen.

Here, halogen is preferably chlorine, bromine, or fluorine, and more preferably chlorine.

A preferable example of aryl is phenyl, and a preferable example of 5- to 6-membered ring heteroaryl is aryl having one or two atoms selected from oxygen, sulfur, and nitrogen; the atoms may be the same or different. Preferably, the aryl is 5- to 6-membered ring aryl having one oxygen as a heteroatom. A preferable example thereof is furyl, and specific examples thereof include furan-1-yl, furan-2-yl, furan-3-yl, furan-4-yl, and furan-5-yl. More preferable is furan-3-yl. Examples of the substituents of aryl and heteroaryl are as described above, and preferable are halogen, C₁₋₆ (preferably C₁₋₄) alkyl, and C₁₋₆ (preferably C₁₋₄) alkoxy. Among these, Cₗ-₄ alkyl is preferable, and more preferable are methyl and ethyl.

R₃ is as defined above, and is preferably hydrogen.

D is as defined above, and preferable examples thereof include aryl optionally having one or two substituents, heteroaryl optionally having one or two substituents, C₃₋₈ cycloalkyl optionally having one or two substituents, C₃₋₈ cycloalkenyl optionally having one or two substituents, and adamanthyl. Here, aryl is preferably phenyl; heteroaryl is preferably furyl, and more preferably furan-2-yl or furan-3-yl; cycloalkyl is preferably cyclohexyl; cycloalkenyl is preferably cyclohexanyl, and more preferably cyclohex-1-en-1-yl; and adamanthyl is preferably adamantan-1-yl.

Examples of the substituents are as described above, and preferable are C₁₋₆ (preferably C₁₋₄) alkyl and C₁₋₆ (preferably C₁₋₄) alkoxy.

Specific examples of the aromatic carboxylic acid (benzenecarboxylic acid) of the present invention represented by Formula (I-c) or bioisosteres of the carboxylic acid include the following compounds shown in Tables 1 to 6:
Example 13, Example 15, Example 16, Example 17, Example 18, Example 19, Example 20, Example 21, Example 22, Example 25, Example 26, Example 27, Example 28, Example 29, Example 30, Example 44, Example 45, Example 46, Example 47, Example 49, Example 50, Example 53, Example 54, Example 55, Example 59, and Example 61.

### (I-d) Compound Wherein L Is Alkylene, Alkenylene, or Alkynylene

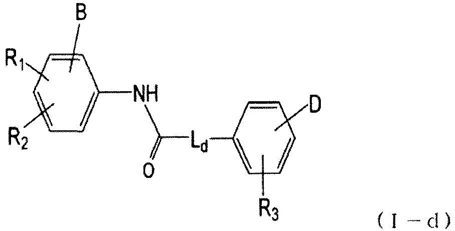

wherein R₁ to R₃, B, and D are as defined above, and L_{d} is substituted or unsubstituted C₁₋₆-alkylene, C₂₋₆-alkenylene, or C₂₋₆-alkynylene.

The alkylene is preferably C₁₋₄ alkylene, and more preferably C₁₋₃ alkylene. The alkylene may be linear or branched, and some carbon atoms in the alkylene optionally form a C₃₋₈ cycloalkyl ring. The cycloalkyl ring (cycloalkane) is preferably cyclopropane.

The alkenylene is preferably C₂₋₃ alkenylene, and more preferably vinylene. The alkynylene is preferably C₂₋₃ alkynylene, and more preferably C₂ alkynylene. These groups may have one or two substituents. Such substituents are as defined above, and preferable examples thereof include unsubstituted alkylene, alkenylene, and alkynylene.

In Formula (I-d) , B, R₁, and R₂ may be located at any of the ortho, meta, and para positions of the benzene ring to which imino is bound. Preferable compounds are those in which B is located at the ortho position of the benzene ring, and R₁ and R₂ are located at the meta and para positions, respectively. R₁ and R₂ are as defined above, preferably are the same or different, and each represents hydrogen or halogen. More preferably, R₁ located at the meta position is hydrogen, and R₂ located at the para position is halogen. The halogen is preferably chlorine, bromine, or fluorine, and more preferably chlorine.

R₃ is as defined above, and is preferably hydrogen.

D is as defined above, and preferable examples thereof include aryl optionally having one or two substituents, heteroaryl optionally having one or two substituents, benzo-fused heteroaryl optionally having one or two substituents, C₃₋₈ cycloalkenyl optionally having one or two substituents, and adamanthyl optionally having one or two substituents.

The aryl is preferably phenyl. Examples of heteroaryl include 5- or 6-membered ring aryl having oxygen or nitrogen as a heteroatom. Preferable are furyl and pyridyl, and more preferable are furan-2-yl, furan-3-yl, and pyridin-3-yl. The benzo-fused heteroaryl is preferably quinolyl or isoquinolyl, and more preferably quinolin-8-yl, quinolin-3-yl, or quinolin-5-yl. The cycloalkenyl is preferably cyclohexanyl, and more preferably cyclohex-1-en-1-yl. The adamanthyl is preferably adamantan-1-yl. Examples of the substituents are as described above, and preferable are unsubstituted aryl, heteroaryl, benzo-fused heteroaryl, cycloalkenyl, and adamanthyl.

Specific examples of the aromatic carboxylic acid (benzenecarboxylic acid) of the present invention represented by Formula (I-d) or bioisosteres thereof include the following compounds shown in Tables 1 to 6:
Example 3, Example 35, Example 36, Example 37, Example 70, Example 78, Example 89, Example 90, Example 101, and desalted products thereof, Example 103 and desalted products thereof, and Example 94.

### (I-e) Compound Wherein L Is Adamantylene, 1,4-Piperazidinyl, or Alkylene-1,4-Piperazidinyl

Preferred among these compounds is a compound represented by Formula (I-e₁) below, wherein L is adamantylene: 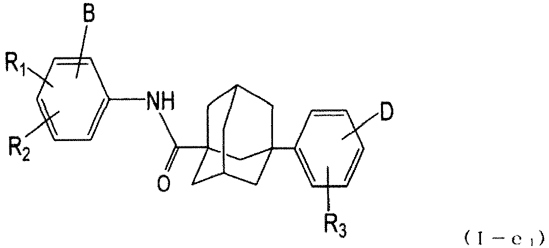 wherein R₁ to R₃, B, and D are as defined above.

In the formula, B, R₁, and R₂ may be located at any of the ortho, meta, and para positions of the benzene ring to which imino is bound. Preferable compounds are those in which B is located at the ortho position of the benzene ring, and R₁ and R₂ are located at the meta and para positions, respectively. R₁ and R₂ are as defined above, preferably are the same or different, and each represents hydrogen or halogen. More preferably, R₁ located at the meta position is hydrogen, and R₂ located at the para position is halogen. The halogen is preferably chlorine, bromine, or fluorine, and more preferably chlorine.

R₃ is as defined above, and is preferably hydrogen.

D is as defined above, and is preferably C₁₋₆ alkyl. C₁₋₄ alkyl is preferable, and C₁₋₂ alkyl is more preferable.

Specific examples of the aromatic carboxylic acid (benzene carboxylic acid) of the present invention represented by Formula (I-e) and bioisosteres thereof include the compound of Example 86 shown in Table 5.

The method for producing the compound group 1 represented by Formula (I) is as described in the international publication pamphlet (WO2010/113022), and these compounds can be produced according to this method.

The following Tables 1 to 7 show the inhibitory activity of the compounds represented by Formula (I) and known compounds having a PAI-1 inhibitory effect (existing compounds 1 to 4) on human PAI-1. Table 1 shows the results measured by the method shown in Reference Experimental Example 1 described later, and Tables 2 to 7 show the results measured by the method shown in Reference Experimental Example 2 described later.

**Table 1**

| Example of compound group 1 | Chemical name | Formula M.W. | PAI-1 activity. % | |
|---|---|---|---|---|
| | | | 50 µM | 20 µM |
| 1 | 5-chloro-2-{[4-({[3-(furan-3-yl)phenyl]carbonyl}amino)butanoyl]amino}benzoic acid | C22H19ClN2O5 426.85 | 48.9 | 95.5 |
| 2 | 5-chloro-2-({[3-(furan-3-yl)phenyl]carbonyl}amino)benzoic acid | C18H12ClNO4 341.75 | 8.7 | 49.0 |
| 3 | 5-chloro-2-({[3-(furan-3-yl)phenyl]acetyl}amino)benzoic acid | C19H14ClNO4 355.77 | 19.6 | 63.7 |
| 4 | 2-[(biphenyl-3-ylcarbonyl)amino]-5-chlorobenzoic acid | C20H14ClNO3 351.78 | 7.6 | 47.7 |
| 5 | 2-{{[3-(furan-3-yl)phenyl]carbonyl}amino)-5-methyl-4-phenylthiophene-3-carboxylic acid | C23H17NO4S 403.45 | 6.1 | 14.7 |
| 6 | 2-[(biphenyl-2-ylcarbonyl)amino]-5-chlorobenzoic acid | C20H14ClNO3 351.78 | 22.2 | 51.4 |
| 7 | 5-chloro-2-({[4-(thiophen-2-yl)phenyl]carbonyl}amino)benzoic acid | C18H12ClNO3S 357.81 | 7.4 | 54.2 |
| 9 | 5-chloro-2-[(1-{[3-(furan-3-yl)phenyl]acetyl}-L-prolyl)amino]benzoic acid | C24H21ClN2O5 452.89 | 53.2 | 98.1 |
| 10 | 5-chloro-2-[(1-{[3-(furan-3-yl)phenyl]carbonyl}-L-prolyl)amino]benzoic acid | C23H19ClN2O5 438.86 | 38.3 | 73.0 |
| 11 | 5-chloro-2-{[(1-{[3-(furan-3-yl)phenyl]carbonyl}piperidin-3-yl)carbonyl]amino}sodium benzoate | C24H20ClN2NaO5 474.87 | 26.1 | 68.2 |
| 12 | 5-chloro-2-{[(1-{[3-(furan-3-yl)phenyl}acetyl}piperidin-3-yl)carbonyl]amino}benzoic acid | C25H23ClN2O5 466.91 | 15.7 | 57.7 |
| 13 | 5-chloro-2-({[3-(furan-3-yl)phenoxy]acetyl}amino)benzoic acid | C19H14ClNO5 371.77 | 8.2 | 39.9 |
| 14 | 5-chloro-2-({[5-(furan-3-yl)-1-methyl-1H-indol-3-yl](oxo)acetyl}amino)benzoic acid | C22H15ClN2O5 422.82 | 12.9 | 22.1 |
| Existing compound 1 | 2-{[(4-*tert-*butylphenyl)carbonyl]amino}-5-chlorobenzoic acid | C18H18ClNO3 331.79 | 11.0 | 44.0 |
| Existing compound 2 | 2-[(biphenyl-4-ylcarbonyl)amino]-5-chlorobenzoic acid | C20H14ClNO3 351.78 | 8.0 | 41.8 |

**Table 2**

| Example of compound group 1 | Chemical name | Formula M.W. | PAI-1 activity: % | |
|---|---|---|---|---|
| | | | 10 µM | 2.5 µM |
| 2 | 5-chloro-2-({[3-(furan-3-yl)phenyl]carbonyl}amino)benzoic acid | C18H12ClNO4 341.75 | 12.7 | 100.6 |
| 4 | 2-[(bipheny)-3-ylcarbonyl)amino]-5-chlorobenzoic acid | C20H14ClNO3 351.78 | 0.6 | 62.1 |
| 5 | 2-({[3-{furan-3-yl)phenyl]carbonyl}amino}-5-methyl-4-phenylthiophene-3-carboxylic acid | C23H17NO4S 403.45 | 0.3 | 46.4 |
| 7 | 5-chloro-2-({[4-(thiophen-2-yl)phenyl]carbonyl}amino)benzoic acid | C18H12ClNO3S 357.81 | 0.4 | 99.8 |
| 8 | 5-chloro-2-({[3-(pyridin-4-yl)phenyl]carbonyl}amino)sodium benzoate | C19H12ClN2NaO3 374.75 | 28.5 | 100.3 |
| 13 | 5-chlono-2-({[3-(furan-3-yl)phenoxy]acetyl}amino)benzoic acid | C19H14ClNO5 371.77 | 4.9 | 92.7 |
| 14 | 5-chloro-2-({[5-(furan-3-yl}-1-methyl-1H-indol-3-yl](oxo)acetyl}amino)benzoic acid | C22H15ClN2O5 422.82 | 4.3 | 87.4 |
| 15 | 5-bromo-2-({[3-(furan-3-yl)phenoxy]acetyl}amino)benzoic acid | C19H14BrNO5 416.22 | 3.0 | 87.3 |
| 16 | 2-{[(3-tert-buty)phenoxy)acetyl]amino}-5-chlorobenzoic acid | C19H20ClNO4 361.82 | 21.9 | 77.4 |
| 17 | 5-chloro-2-{[(2-cydohexy)phenoxy)acety)]amino}benzoic acid | C21H22ClNO4 387.86 | 0.8 | 52.8 |
| 18 | 2-{[(4-tert-butylphenoxy)acetyl]amino}-5-chlorobenzoic acid | C19H20CIN04 361.82 | 16.1 | 81.4 |
| 19 | 2-{[(bipheny)-4-yloxy)acetyl]amino}-5-chlorobenzoic acid | C21H16ClNO4 381.81 | 2.5 | 72.4 |
| 20 | 2-{[(bipheny)-3-yloxy)acetyl]amino}-5-chlorobenzoic acid | C21H16CINO4 381.81 | 1.9 | 55.7 |
| 21 | 2-({[4-(adamantan-1-yl)phenoxy]acetyl}amino)-5-chlorobenzoic acid | C25H26ClNO4 439.93 | 0.3 | 10.0 |
| 22 | 4-({[3-(furan-3-yl)phenoxy]acetyl}amino)biphenyl-3-carboxylic acid | C25H19NO5 413.42 | 0.4 | 29.9 |
| 23 | 2-{[(5-bromo-1-methyl-1H-indol-2-yl)carbonyl]amino}-5-chlorobenzoic acid | C17H12BrClN2O3 407.65 | 1.8 | 97.3 |
| 24 | 2-{[(5-bromo-1H-indol-1-yl)acetyl]amino}-5-chlorobenzoic acid | C17H12BrClN2O3 407.65 | 19.1 | 101.5 |
| 25 | 5-chloro-2-({[3-(cyclohex-1-en-1-yl)phenoxy]acetyl}amino)benzoic acid | C21H20ClNO4 385.84 | 0.0 | 85.8 |
| 26 | 5-chloro-2-{[(3-cyclohexylphenoxy)acetyl]amino}benzoic acid | C21H22ClNO4 387.86 | 0.6 | 98.0 |
| 27 | 4-({[3-(furan-3-yl)phenoxy]acetyl}amino)-3'-methylbiphenyl-3-carboxylic acid | C26H21NO5 427.45 | 0.7 | 69.1 |

**Table 3**

| Example of compound group 1 | Chemical name | Formula | M.W. | PAI-1 activity: % | |
|---|---|---|---|---|---|
| | | | | 10 µM | 2.5 µM |
| 28 | 4-({[3-(furan-3-yl)phenoxy]acetyl}amino-3',5'-dimethylbiphenyl-3-carboxylic acid | C27H23NO5 | 441.48 | 0.5 | 33.6 |
| 29 | 5-chloro-2-({[4-(furan-3-yl)phenoxy]acetyl}amino)benzoic acid | C19H14ClNO5 | 371.77 | 24.3 | 93.2 |
| 30 | 2-({[3-(adamantan-1-yl)phenoxy]acetyl}amino)-5-chlorobenzoic acid | C25H26ClNO4 | 439.93 | 1.3 | 19.3 |
| 31 | 2-{{[1-{biphenyl-3-ylcarbonyl)piperidin-3-yl]carbonyl}amino}-5-chlorobenzoic acid | C26H23ClN2O4 | 462.92 | 20.2 | 88.0 |
| 32 | 5-chloro-2-{[(4'-methylbiphenyl-3-yl)carbonyl]amino}benzoic acid | C21H16ClNO3 | 365.81 | 1.7 | 94.4 |
| 33 | 5-chloro-2-{[(2'-methoxybipheny)-3-yl)carbonyl]amino}benzoic acid | C21H16ClNO4 | 381.81 | 21.6 | 92.8 |
| 34 | 5-chloro-2-({[4-(3,6-dihydro-2H-pyran-4-y))phenyl]carbonyl}amino)benzoic acid | C19H16ClNO4 | 357.79 | 45.1 | 97.1 |
| 35 | 5-chloro-2-[({1-[3-(furan-3-yl)phenyl]cyclopropyl}carbonyl)amino]benzoic acid | C21H16ClNO4 | 381.81 | 15.6 | 98.8 |
| 36 | 5-chloro-2-({3-[3-(furan-3-yl)phenyl]propanoyl}amino)benzoic acid | C20H16ClNO4 | 369.80 | 3.7 | 95.7 |
| 37 | 5-chloro-2-({2-[3-(furan-3-yl)phenyl]-methylpropanoyl}amino)benzoic acid | C21H18ClNO4 | 383.82 | 5.9 | 53.1 |
| 38 | 5-chloro-2-[(9H-fluoren-1-ylcarbonyl)amino]benzoic add | C21H14ClNO3 | 363.79 | 2.5 | 94.8 |
| 39 | 5-chloro-2-[(2,2-diphenylpropanoyl)amino]benzoic acid | C22H18ClNO3 | 379.84 | 22.1 | 88.6 |
| 40 | 2-({[4-(adamantan-1-yl)phenyl]carbonyl]amino)-5-chlorobenzoic acid | C24H24ClNO3 | 409.91 | 1.7 | 19.3 |
| 41 | 5-chloro-2-[(3,3-diphenylpropanoyl)amino]benzoic acid | C22H18ClNO3 | 379.84 | 12.9 | 72.4 |
| 42 | 5-chloro-2{[(4-phenoxyphenyl)carbonyl]amino}benzoic acid | C20H14ClNO4 | 367.78 | 3.8 | 98.6 |
| 43 | 2-({[3,5-bis(trifluoromethyl)phenyl]carbonyl]amino)-5-chbrobenzoic acid | C16H8ClF6NO3 | 411.68 | 14.1 | 98.4 |
| 44 | 5-chloro-2-({2-[3-(furan-3-yl)phenoxy]-2-methylpropanoyl}amino)benzoic acid | C21H18ClNO5 | 399.82 | 20.4 | 97.8 |
| 45 | 4-{[biphenyl-3-yloxy)acetyl]amino}biphenyl-3-carboxylic acid | C27H21NO4 | 423.46 | 0.9 | 76.9 |
| 46 | 2-{[(biphenyl-4-yloxy)acetyl]amino}-5-(furan-3-yl)benzoic acid | C25H19NO5 | 413.42 | 0.4 | 69.9 |
| 47 | 2-({[4-(adamantan-1-yl)phenoxy)acetyl}amino}-5-(furan-3-yl)benzoic acid | C29H29NO5 | 471.54 | 0.6 | 9.7 |
| 48 | 5-chloro-2-{[(1-methyl-5-phenyl-1H-indol-2-yl)carbonyl]amino}benzoic acid | C23H17ClN2O3 | 404.85 | 1.7 | 2.5 |

**Table 4**

| Example of compound group 1 | Chemical name | Formula | M.W. | PAI-1 activity: % | |
|---|---|---|---|---|---|
| | | | | 10 µM | 2.5 µM |
| 49 | 5-chloro-2-({[(4'-methylbiphenyl-4-yl)oxy]acetyl}amino)benzoic acid | C22H18ClNO4 | 395.84 | 1.1 | 82.1 |
| 50 | 5-chloro-2-({[(3',5'-dimethylbiphenyl-4-yl)oxy]acetyl}amino)benzoic acid | C23H20ClNO4 | 409.86 | 0.1 | 58.9 |
| 51 | 5-chloro-2-{[(5-phenyl-1H-indol-1-yl)acetyl]amino}benzoic acid | C23H17ClN2O3 | 404.85 | 1.7 | 37.8 |
| 52 | 2-{{[4-(adamantan-1-ylmethoxy)phenyl]carbonyl}amino)-5-chlorobenzoic acid | C25H26ClNO4 | 439.93 | 5.1 | 7.2 |
| 53 | 5-chloro-2-({[3-(furan-2-yl)phenoxy]acetyl}amino)benzoic acid | C19H14ClNO5 | 371.77 | 21.6 | 95.3 |
| 54 | 5-chloro-2-({[4-(furan-2-yt)phenoxy]acetyl}amino)benzoic acid | C19H14ClNO5 | 371.77 | 15.5 | 94.4 |
| 55 | 2-({4-{4-(adamantan-1-yl)phenoxy]butanoyl}amino)-5-chlorobenzoic acid | C27H30ClNO4 | 467.98 | 1.1 | 29.8 |
| 56 | 2-({[4-(adamantan-1-y)carbonyl)phenyl]carbonyl}amino}-5-chlorobenzoic acid | C25H24ClNO4 | 437.92 | 0.7 | 75.8 |
| 57 | 2-{{[5-(benzy)oxy)-1H-indol-3-yl](oxo)acetyl}amino}-5-chlorobenzoic acid | C24H17ClN2O5 | 448.85 | 0.2 | 38.6 |
| 58 | 5-chloro-2-({[3-(naphthalen-1-yl)phenyl]carbonyl}amino)benzoic acid | C24H16ClNO3 | 401.84 | 0.0 | 0.0 |
| 59 | 2-({3-[4-(adamantan-1-y))phenoxy]propanoyl}amino)-5-chlorobenzoic acid | C26H28ClNO4 | 453.96 | 1.4 | 56.3 |
| 60 | 5-chloro-2-({[1-(3-hydroxypropyl)-5-phenyl-1H-indol-2-yl]carbonyl}amino)benzoic acid | C25H21ClN2O4 | 448.90 | 0.9 | 25.0 |
| 61 | 5-chloro-2-({[(2'-methoxybiphenyl-3-yl]acetyl}amino)benzoic acid | C22H18ClNO5 | 411.84 | 38.9 | 92.4 |
| 62 | 2-({[3-(adamantan-1-yl)phenyl]carbonyl}amino)-5-chlorobenzoic acid | C24H24ClNO3 | 409.91 | 0.6 | 29.1 |
| 63 | 5-chloro-2-({[3-(quinolin-3-yl)phenyl]carbonyl}amino)sodium benzoate | C23H14ClN2NaO3 | 424.81 | 0.3 | 21.3 |
| 64 | 5-chloro-2-({[3-(isoquinolin-4-yl)phenyl]carbonyl}amino)sodium benzoate | C23H14ClN2NaO3 | 424.81 | 0.7 | 74.3 |
| 65 | 5-chloro-2-({[3-(quinolin-6-yl)phenyl]carbonyl}amino)sodium benzoate | C23H14ClN2NaO3 | 424.81 | 0.4 | 71.7 |
| 66 | 5-chloro-2-({[3-(isoquinolin-5-yl)phenyl]carbonyl}amino)sodium benzoate | C23H14CIN2Na03 | 424.81 | 0.1 | 22.6 |
| 67 | 5-chloro-2-({[4-(quinolin-8-yl)phenyl]carbonyl}amino)sodium benzoate | C23H14ClN2NaO3 | 424.81 | 0.6 | 90.4 |
| 68 | 5-chloro-2-({[3-(quinolin-8-yl)phenyl]carbonyl}amino)sodium benzoate | C23H14ClN2NaO3 | 424.81 | 2.1 | 2.4 |

**Table 5**

| Example of compound group 1 | Chemical name | Formula | M.W. | PAI-1 activity: % | |
|---|---|---|---|---|---|
| 69 | 5-chloro-2-{[(4-cyclohexylphenyl)carbonyl]amino)benzoic acid | C20H20ClNO3 | 357.83 | 0.6 | 44.7 |
| 70 | 2-[(biphenyl-4-ylacetyl)amino]-5-chlorobenzoic acid | C21H16ClNO3 | 365.81 | 4.4 | 94.6 |
| 71 | 2-[(biphenyl-4-ylcarbamoyl)amino]-5-chlorobenzoic acid | C20H15ClN2O3 | 366.80 | 2.1 | 39.6 |
| 72 | 5-chloro-2-{[N-(4'-fluoro-4-methylbiphenyl-3-yl)glycyl]amino}benzoic acid | C22H18ClFN2O3 | 412.84 | 3.6 | 31.1 |
| 73 | 5-chloro-2-{[N-(diphenylmethyl)glycyl]amino}benzoic acid | C22H19ClN2O3 | 394.85 | 31.2 | 82.2 |
| 74 | 2-{[(4-cyclohexylphenyl)carbonyl]amino}-5-methyt-4-phenylthiophene-3-carboxylic acid | C25H25NO3S | 419.54 | 0.2 | 8.6 |
| 75 | 5-chloro-2-({[4-(diphenylmethyl)piperazin-1-yl]carbonyl}amino)benzoic acid hydrochloride | C25H25Cl2N3O3 | 486.39 | 4.7 | 86.4 |
| 76 | 5-chloro-2-{[(diphenylmethoxy)acetyl]amino}benzoic acid | C22H18ClNO4 | 395.84 | 30.6 | 92.9 |
| 77 | 5-chloro-2-({[4-(diphenylmethyl)piperazin-1-yl]acetyl}amino)benzoic acid | C26H26ClN3O3 | 463.96 | 19.3 | 89.6 |
| 78 | 2-{[(2E}-3-(biphenyl-4-yl)prop-2-enoyl]amino}-5-chlorobenzoic acid | C22H16ClNO3 | 377.82 | 0.3 | 25.3 |
| 79 | 5-chloro-2-({[3-(cyclohex-1-en-1-yl)pheny)]carbony)}amino)benzoic acid | C20H18ClNO3 | 355.81 | 1.6 | 44.5 |
| 80 | 5-chloro-2-{[(3-cyclohexylphenyl)carbonyl]amino}benzoic acid | C20H20ClNO3 | 357.83 | 0.0 | 45.5 |
| 81 | 5-(cyclohex-1-en-1-yl)-2-{[(4-cyclohexylphenyl)carbonyl]amino}benzoic acid | C26H29NO3 | 403.51 | 0.0 | 1.0 |
| 82 | 5-cyclohexyl-2-([(4-cyclohexylphenyl)carbonyl]amino}benzoic acid | C26H31NO3 | 405.53 | 1.4 | 1.7 |
| 83 | 5-chloro-2-({[4-(pyrrolidin-1-yl)phenyl]carbonyl}amino)benzoic acid hydrochloride | C18H18Cl2N2O3 | 381.25 | 26.6 | 99.1 |
| 84 | 5-chloro-2-[(spiro[5.5]undec-1-en-2-ylcarbonyl)amino]benzoic acid | C19H22ClNO3 | 347.84 | 5.5 | 97.0 |
| 85 | 5-chloro-2-[(spiro[5.5]undec-2-ylcarbonyl)amino]benzoic acid | C19H24ClNO3 | 349.85 | 11.0 | 97.5 |
| 86 | 5-chloro-2-({[3-(4-methylphenyl)adamantan-1-yl]carbonyl}amino)benzoic acid | C25H26ClNO3 | 423.93 | 1.4 | 63.5 |
| 87 | 5-chloro-2-({[3-(cyclohexylethynyl)phenyl]carbonyl}amino)benzoic acid | C22H20ClNO3 | 381.85 | 1.2 | 20.5 |
| 88 | 5-chloro-2-({[4-(cyclohexylethynyl)phenyl]carbonyl}amino)benzoic acid | C22H20ClNO3 | 381.85 | 0.8 | 35.5 |
| 89 | 2-{[(2E)-3-(biphenyl-3-yl)prop-2-enoyl]amino}-5-chlorobenzoic acid | C22H16ClNO3 | 377.82 | 0.1 | 60.7 |

**Table 6**

| Example of compound group 1 | Chemical name | Formula | M.W. | PAI-1 | activity: % |
|---|---|---|---|---|---|
| 90 | 5-chloro-2-({(2E)-3-[3-(cyclohex-1-en-1-yl)phenyl]prop-2-enoyl}amino)benzoic acid | C22H20ClNO3 | 381.85 | 0.1 | 65.3 |
| 91 | 5-chloro-2-{[(2E)-3-chlro-3-cyclohexylprop-2-enoyl]amino}benzoic acid | C16H17Cl2NO3 | 342.22 | 16.7 | 96.9 |
| 92 | 5-{cyclohexylethynyl)-2-{[(4-cyclohexylphenyl)carbony)]amino}benzoic acid | C28H31NO3 | 429.55 | 0.4 | 0.0 |
| 93 | 5-(2-cyclohexylethyl)-2-{[(4-cyclohexylphenyl)carbonyl]amino}benzoic acid | C28H35NO3 | 433.58 | 0.0 | 0.0 |
| 94 | 2-({3-{4-(adamantan-1-yl)phenyl]prop-2-enoyl}amino)-5-chlorobenzoic acid | C26H24ClNO3 | 433.93 | 7.4 | 3.5 |
| 95 | 2-{[(4-cyclohexylphenyl)carbonyl]amino}-5-ethynylbenzoic acid | C22H21NO3 | 347.41 | 0.3 | 80.5 |
| 96 | 2-({[4-(adamantan-1-y)methyl)phenyl)carbonyl}amino)-5-chlorobenzoic acid | C25H26ClNO3 | 423.93 | 5.0 | 23.9 |
| 97 | 2-[({4-[adamantan-1-yl(hydroxy)methy)]phenyl}carbonyl)amino]-5-chlorobenzoic acid | C25H26ClNO4 | 439.93 | 0.7 | 89.9 |
| 98 | 5-chloro-2-({[4-(1-methylcyclohexyl)phenyl]carbonyl)amino)benzoic acid | C21H22ClNO3 | 371.86 | 0.1 | 41.5 |
| 99 | 5-chloro-2-({[3-(quinolin-2-ylmethoxy)phenyl]carbonyl}amino)sodium benzoate | C24H16ClN2NaO4 | 454.84 | 0.3 | 87.8 |
| 100 | 5-chloro-2-({[4-(quinolin-2-ylmethoxy)phenyl]carbonyl}amino)sodium benzoate | C24H16ClN2NaO4 | 454.84 | 0.7 | 92.1 |
| 101 | 5-chloro-2-({(2E)-3-[3-(quinolin-8-yl)phenyl]prop-2-enoyl}amino)sodium benzoate | C25H16ClN2NaO3 | 450.85 | 0.2 | 24.7 |
| 102 | N-[4-chloro-2-(1H-tetrazol-5-yl)phenyl]-3-(quinolin-8-yl)benzamide | C23H15ClN6O | 426.86 | 1.5 | 91.3 |
| 103 | 5-chloro-2-({(2E)-3-[3-(pyridin-3-yl)phenyl]prop-2-enoyl}amino)sodium benzoate | C21H14ClN2NaO3 | 400.79 | 34.2 | 92.3 |
| 104 | 5-chloro-2-({[5-(fluorophenyl)thiophen-2-yl]carbonyl}amino)benzoic acid | C18H11ClFNO3S | 375.80 | 0.9 | 84.8 |
| 105 | 5-chloro-2-{[(5-phenylfuran-2-yl)carbonyl]amino}benzoic acid | C18H12ClNO4 | 341.75 | 23.5 | 89.9 |
| 106 | 2-{({[4-(adamantan-1-yl)phenyl)amino}(oxo)acetyl)amino}-5-sodium chlorobenzoate | C25H24ClN2NaO4 | 474.91 | 26.3 | 88.4 |
| 107 | 5-chloro-2{[(2-phenylquinolin-4-yl)carbonyl}amino}sodium benzoate | C23H14ClN2NaO3 | 424.81 | 6.7 | 100.0 |

**Table 7**

| Example of existing compound | Chemical name | Formula M.W. | | PAI-1 activity: % | |
|---|---|---|---|---|---|
| Existing compound 1 | 2-{[(4-tert-buty)phenyl)carbonyl]amino}-5-chlorobenzoic acid | C18H18ClNO3 | 331.79 | 12.7 | 100.5 |
| Existing compound 2 | 2-[(biphenyl-4-ylcarbonyl)amino]-5-chlorobenzoic acid | C20H14ClNO3 | 351.78 | 0.2 | 70.7 |
| Existing compound 3 | 5-chloro-2-{[(4-cycbhexylphenoxy)acetyl]amino}benzoic acid | C21H22ClNO4 | 387.86 | 0.3 | 36.3 |
| Existing compound 4 | 5-chloro-2-({[4-(1H-pyerol-1-yl)phenyl]carbonyl}amino)benzoic acid hydrochloride | C18H14Cl2N2O3 | 377.22 | 3.7 | 100.1 |

Each of the compounds (I) targeted by the present invention may be in free, salt, or ester form.

Examples of salts as used herein typically include pharmaceutically acceptable salts, e.g., a salt formed with an inorganic base or organic base, a salt formed with a basic amino acid, and other salts. Examples of inorganic bases include alkali metals such as sodium, potassium, etc.; alkaline earth metals such as calcium, magnesium, etc.; and aluminum, ammonium, etc. Examples of organic bases include primary amines such as ethanolamine, tromethamine, ethylenediamine, etc.; secondary amines such as diethylamine, diethanolamine, meglumine, dicyclohexylamine, N,N'-dibenzylethylenediamine, etc.; and tertiary amines such as trimethylamine, triethylamine, pyridine, picoline, triethanolamine, etc. Examples of basic amino acids include arginine, lysine, ornithine, histidine, etc. Further, the compound of the present invention may form a salt with an inorganic acid or organic acid. Examples of inorganic acids include hydrochloric acid, hydrobromic acid, sulfuric acid, phosphoric acid, etc. Examples of organic acids include formic acid, acetic acid, trifluoroacetic acid, maleic acid, tartaric acid, fumaric acid, citric acid, lactic acid, methanesulfonic acid, benzenesulfonic acid, toluenesulfonic acid, etc.

Examples of esters as used herein typically include pharmaceutically acceptable esters, such as alkoxycarbonyl, aryloxycarbonyl, and aralkyloxycarbonyl, which can be converted to carboxyl when absorbed *in vivo.* In particular, specific examples of the "alkoxycarbonyl" represented by B in Formula (I) include t-butoxycarbonyl, methoxycarbonyl, ethoxycarbonyl, propoxycarbonyl, isopropoxycarbonyl, butoxycarbonyl, etc.

Further, when the carboxylic acid represented by Formula (I), a bioisostere of the carboxylic acid, or a salt or ester thereof forms a solvate (e.g., hydrate, alcoholate), such a solvate is also encompassed in the present invention. Furthermore, the present invention encompasses all of the compounds (e.g., so-called prodrugs) that are converted, when metabolized *in vivo,* to a carboxylic acid represented by Formula (I), a bioisostere thereof, or a pharmaceutically acceptable salt or ester thereof.

Further, the compound having PAI-1 inhibitory activity (PAI-1 inhibitor) targeted by the present invention includes, in addition to the compound group 1 including the compound (I) described above, and the compound groups 2 and 3, known compounds that are known to have a PAI-1 inhibitory effect (PAI-1 inhibitors).

The PAI-1 inhibitors include direct PAI-1 inhibitors that directly interact with or bind to PAI-1 to reduce the activity of PAI-1, and indirect PAI-1 inhibitors that indirectly inhibit (suppress or downregulate) the activity of PAI-1.

Examples of direct PAI-1 inhibitors include, but are not limited to, the various compounds described in paragraphs [0042] to [0043] of JP2006-507297A. The description in this publication is incorporated herein by reference. Direct PAI-1 inhibitors also include, as described in this publication, in addition to various low-molecular-weight compounds, antibodies such as PAI-1 neutralizing antibodies and polyclonal or monoclonal antibodies against the reactive central loop (Ser333-Lys346 of PAI-1), and fragments and derivatives thereof (e.g., Fab fragment, scFv, minibodies, and diabodies). Other anti-PAI-1 antibodies include the human anti-PAI-1 antibody described in WO2015/125904 and the anti-PAI-1 antibody described in JP2016-529255A (the descriptions in these publications are also incorporated herein by reference). Further, it is also known to inactivate PAI-1 activity by inhibiting intramolecular interactions of the glycine residue at position 374 of PAI-1 and its neighboring region (JP2015-147744A); thus, compounds that can inhibit intramolecular interactions can also be used as direct PAI-1 inhibitors (the description in this publication is also incorporated herein by reference).

Further, examples of indirect PAI-1 inhibitors include, but are not limited to, the various compounds described in paragraphs [0044] to [0046] of JP2006-507297A. The description in this publication is incorporated herein by reference. Specifically, compounds that specifically inhibit the transcription or expression of PAI-1 gene at the cellular and molecular level, antisense that blocks the expression of PAI-1 gene (e.g., antisense oligonucleotide complementary to the PAI-1 sequence), siRNAs that degrade mRNA of PAI-1, dicers that produce such siRNAs, and compounds that compete with PAI-1 in enzymatic reactions with a plasminogen activator.

The expression of PAI-1 is known to be enhanced, for example, by endotoxin, thrombin, TNF-α, TGF-β, interleukin-1, insulin, angiotensin II, dexamethasone, PDGF, EGF, or the like. Therefore, for example, angiotensin-converting enzyme inhibitors (e.g., fosinopril, imidapril, captopril, and enalapril) and angiotensin II receptor antagonists (e.g., L158809 and eprosartan) can be used as indirect PAI-1 inhibitors that inhibit or weaken the expression of PAI-1.

The "PD-L1 expression inhibitor," "immunostimulator," "immune checkpoint inhibitor (ICI)," "inhibitor for exacerbation of tumor cells caused by PD-L1 (tumor cell exacerbation inhibitor)," "tumor chemotherapy agent," "enhancer of immunotherapy for tumors," and "pharmaceutical composition" described below all comprise the PAI-1 inhibitor descried above as a sole or main active ingredient. Therefore, the description in section I is incorporated by reference in the following sections II to IV.

### II. PD-L1 Expression Inhibitor, Immunostimulator, Immune Checkpoint Inhibitor (ICI), Tumor Cell Exacerbation Inhibitor, and Immunotherapy Enhancer

All of the PD-L1 expression inhibitor, immunostimulator, ICI, tumor cell exacerbation inhibitor, and immunotherapy enhancer of the present invention comprise a compound having a PAI-1 inhibitory effect (PAI-1 inhibitor) as a sole or main active ingredient.

The PAI-1 inhibitor inhibits the activity of PAI-1, and particularly inhibits the induction of PD-L1 expression by PAI-1 in tumor cells and/or immunosuppressive cells constituting the tumor-surrounding environment, thereby acting to inhibit an immune escape mechanism using PD-L1/PD-1 as an immune checkpoint. For this reason, the PAI-1 inhibitor can be used as a PD-L1 expression inhibitor. The "PD-L1 expression" targeted by the present invention is not limited to the constant expression of PD-L1 on the cell membrane, but also includes transient expression and variable expression. Preferred forms of expression include transient, constant, or variable expression that occurs during the course of tumor development or exacerbation. In addition, the term "inhibition" not only means that the expression of PD-L1 in the above cells is completely eliminated, but also means that the expression of PD-L1 is partially eliminated.

The PAI-1 inhibitor also inhibits immune depression caused by the immune escape mechanism described above and can be used as an immunostimulator.

Further, based on the above mechanism of action, the PAI-1 inhibitor can also be used as an ICI targeting PD-L1.

The ICI targeting PD-L1 refers to a drug that has an action to activate (stimulate) immunity by directly or indirectly inhibiting the function of at least PD-L1 among ICMs, thereby releasing the state of functional suppression of cytotoxic T cells by PD-L1. Due to the immunostimulatory action of the ICI, the original immune system of the living body functions, and the use of the ICI alone or in combination with at least one of other antitumor agents, other immunostimulators, and other ICIs makes it possible to inhibit the proliferation, infiltration, metastasis, or recurrence of tumor cells and to suppress cancer exacerbation.

Here, the "function of PD-L1" includes the activation and expression of PD-L1, preferably the expression of PD-L1 in tumor cells and/or immunosuppressive cells constituting the tumor-surrounding environment. In addition, the term "inhibition" not only means that the activity and expression of PD-L1 are completely eliminated, but also means that the activity and expression of PD-L1 are partially eliminated.

ICIs using the PAI-1 inhibitor as an active ingredient preferably include drugs that have the effect of inhibiting the induction of PD-L1 expression by PAI-1, releasing the state of suppression of cytotoxic T cells, and activating (stimulating) immunity.

Further, based on the above mechanism of action, the PAI-1 inhibitor can also be used alone or in combination with at least one of other antitumor agents, other immunostimulators, and other ICIs, as an enhancer of immunotherapy for tumors or an inhibitor for exacerbation of tumor cells caused by PD-L1. Here, the "exacerbation" of tumor cells includes proliferation, infiltration, metastasis, and/or recurrence of tumor cells caused by PD-L1. Preferably, it is the exacerbation of tumor cells caused by PD-L1 induced by PAI-1. In addition, "inhibition of exacerbation" is not limited to complete blocking of at least one exacerbation phenomenon selected from the proliferation, infiltration, metastasis, and recurrence of tumor cells, but also blocking of some of the exacerbation phenomena, delaying the exacerbation phenomena, and reducing the probability of the occurrence of the exacerbation phenomena. As a result, it can also be advantageously used to improve prognosis after antitumor therapy.

The term "PD-L1 expression inhibitor" is described below as including the meaning as an immunostimulator, the meaning as an ICI, and the meaning as an inhibitor for exacerbation of tumor cells caused by PD-L1 and an enhancer of immunotherapy for tumors.

The "PD-L1 expression inhibitor" of the present invention comprising the PAI-1 inhibitor as an active ingredient can be used for cells and tissues expressing PD-L1, and mammals including humans having them. In particular, when cells expressing PD-L1 are tumor cells (including both hematopoietic organ tumor cells and solid tumor cells), the PD-L1 expression inhibitor of the present invention can be used alone or in combination with at least one member selected from the group consisting of other antitumor agents, other immunostimulators, and other ICIs, as a "tumor chemotherapy agent." This is described in more detail in section III.

The dosage of the PD-L1 expression inhibitor of the present invention can be determined according to the dosage of a tumor chemotherapy agent described below.

### III. Tumor Chemotherapy Agent and Composition for Treating Tumors

The tumor chemotherapy agent and a composition for treating tumors of the present invention each comprise at least the compound having a PAI-1 inhibitory effect described above (PAI-1 inhibitor) as a sole or main active ingredient. Further, the tumor chemotherapy agent and the composition for treating tumors of the present invention may each comprise the PAI-1 inhibitor in combination with at least one member selected from the group consisting of other antitumor agents, other immunostimulators, and other ICIs. The PAI-1 inhibitor as used herein includes those that are used as the "PD-L1 expression inhibitor," "immunostimulator," "ICI," "inhibitor for exacerbation of tumor cells caused by PD-L1," and "enhancer of immunotherapy for tumors" described above. Therefore, the term "PAI-1 inhibitor" referred to below can be paraphrased as the "PD-L1 expression inhibitor," "immunostimulator," "ICI," "tumor cell exacerbation inhibitor," or "enhancer of immunotherapy for tumors."

The tumors targeted by the present invention include tumors that can transiently, constantly, or variably express PD-L1 on the cell membrane in the course of tumor development, progression, or exacerbation. To this extent, examples of such tumors include hematopoietic organ tumors, as well as nonepithelial and epithelial malignant solid tumors. Examples of solid tumors include tumors of the head and neck; glioblastoma (including glioblastoma multiforme); respiratory malignant tumors generated in the bronchi, lung, or the like (including, for example, advanced-stage non-small-cell lung cancer); gastrointestinal tract malignant tumors generated in the epipharynx, esophagus, stomach, duodenum, jejunum, ileum, cecum, appendix, ascending colon, transverse colon, sigmoid colon, large intestine, rectum, anal area, or the like; liver cancer, hepatocellular carcinoma; pancreatic cancer; urinary malignant tumors generated in the bladder, ureter, or kidney (e.g., bladder cancer and renal cell carcinoma); female genital malignant tumors generated in the ovary, oviduct, uterus (uterine cervix, uterine body), or the like; breast cancer; prostatic cancer; skin cancer (including melanoma); malignant tumors in the endocrine system, such as hypothalamus, pituitary gland, thyroid, parathyroid, or adrenal gland; central nervous system malignant tumors; malignant tumors generated in bone soft tissue; and sarcoma. Examples of hematopoietic organ tumors include myelodysplastic syndrome; various leukemias, such as acute lymphocytic leukemia, acute myelogenous leukemia, chronic lymphocytic leukemia, chronic myelogenous leukemia, acute myelomonocytic leukemia, chronic myelomonocytic leukemia, acute monocytic leukemia, chronic monocytic leukemia, acute promyelocytic leukemia, acute megakaryocytic leukemia, erythroleukemia, eosinophilic leukemia, chronic eosinophilic leukemia, chronic neutrophilic leukemia, adult T-cell leukemia, hairy cell leukemia, and plasma cell leukemia; hematopoietic system malignant tumors, such as thymic carcinoma, multiple myeloma, B-cell lymphoma, and malignant lymphoma; and lymph malignant tumors. At least some malignant tumors are known to express PD-L1 (see Sandip Pravin Patel et al., Molecular Cancer Therapeutics, 2015; 14(4); 847-856, Table 1). Just to note, examples of tumor cells on which previously known ICIs, such as anti-PD-1 antibodies, are known to be effective include hematopoietic organ tumors (including, for example, chronic myelogenous leukemia, acute myelogenous leukemia, chronic eosinophilic leukemia, and acute lymphocytic leukemia), ovarian cancer, cervical cancer, endometrial cancer, soft-tissue sarcoma, breast cancer, esophageal cancer, gastric cancer, esophagogastric junction cancer, lung cancer (non-small-cell lung cancer, small-cell lung cancer), hepatocellular carcinoma, renal cell carcinoma, malignant melanoma, Hodgkin's lymphoma, head and neck cancer, malignant pleural mesothelioma, glioblastoma, urothelial carcinoma, bladder cancer, pancreatic cancer, prostate cancer, Merkel cell carcinoma, primary central nervous system lymphoma, primary testicular lymphoma, biliary tract cancer, and colorectal cancer originating from the ascending colon, transverse colon, sigmoid colon, and rectum.

The tumor chemotherapy agent of the present invention includes an embodiment in which the PAI-1 inhibitor is administered alone or in which the PAI-1 inhibitor and at least one member selected from the group consisting of other antitumor agents, other immunostimulators, and other ICIs (collectively referred to below as "the other antitumor agents etc.") are administered simultaneously or separately.

Examples of the other antitumor agents etc. for use in combination with the PAI-1 inhibitor include those generally used as anticancer agents. The other antitumor agents etc. can be suitably selected from alkylating agents, antimetabolites, antitumor antibiotics, microtubule inhibitors, hormones or hormone-like drugs, platinum-based drugs, topoisomerase inhibitors, cytokines, hormone therapy drugs, radioimmunotherapy drugs, molecularly targeted drugs (including antibody drugs and ICIs), non-specific immunostimulants, and other antitumor agents, depending on the target tumor.

Although there is no limitation, examples of alkylating agents include cyclophosphamide, ifosfamide, busulfan, melphalan, bendamustine hydrochloride, nimustine hydrochloride, ranimustine, dacarbazine, procarbazine hydrochloride, temozolomide, etc.; examples of antimetabolites include methotrexate, pemetrexed sodium, fluorouracil, doxifluridine, capecitabine, tegafur, cytarabine, cytarabine ocfosfate hydrate, enocitabine, gemcitabine hydrochloride, mercaptopurine hydrate, fludarabine phosphate, nelarabine, pentostatin, cladribine, calcium levofolinate, calcium folinate, hydroxycarbamide, L-asparaginase, azacytidine, etc.; examples of antitumor antibiotics include doxorubicin hydrochloride, daunorubicin hydrochloride, pirarubicin, epirubicin hydrochloride, idarubicin hydrochloride, aclarubicin hydrochloride, amrubicin hydrochloride, mitoxantrone hydrochloride, mitomycin-C, actinomycin-D, bleomycin, peplomycin sulfate, zinostatin stimalamer, etc.; examples of microtubule inhibitors include vincristine sulfate, vinblastine sulfate, vindesine sulfate, vinorelbine tartrate, paclitaxel, docetaxel hydrate, eribulin mesilate, etc.; examples of hormones or hormone-like drugs (hormonal agents) include anastrozole, exemestane, letrozole, tamoxifen citrate, toremifene citrate, fulvestrant, flutamide, bicalutamide, medroxyprogesterone acetate, estramustine phosphate sodium hydrate, goserelin acetate, leuprorelin acetate, etc.; examples of platinum-based drugs include cisplatin, miriplatin hydrate, carboplatin, nedaplatin, oxaliplatin, etc.; examples of topoisomerase inhibitors include topoisomerase I inhibitors, such as irinotecan hydrochloride hydrate and nogitecan hydrochloride, and topoisomerase II inhibitors, such as etoposide and sobuzoxane; examples of cytokines include interferon gamma-la, teceleukin, celmoleukin, etc.; examples of radioimmunotherapy drugs include ibritumomab, tiuxetan combination drugs, etc.; examples of molecularly targeted drugs include gefitinib, imatinib mesilate, bortezomib, erlotinib hydrochloride, sorafenib tosilate, sunitinib malate, thalidomide, nilotinib hydrochloride hydrate, dasatinib hydrate, lapatinib tosilate hydrate, everolimus, lenalidomide hydrate, dexamethasone, temsirolimus, vorinostat, tretinoin, tamibarotene, etc.; of these, examples of antibody drugs include trastuzumab (anti-HER2 antibody), rituximab (anti-CD20 antibody), bevacizumab (anti-VEGFR antibody), panitumumab and cetuximab (both anti-EGFR antibodies), ramucirumab (anti-VEGFR antibody), mogamulizumab (anti-CCR4 antibody), brentuximab vedotin (anti-CD30 antibody), alemtuzumab (anti-CD52 antibody), gemtuzumab ozogamicin (anti-CD33 antibody), etc.; examples of immune checkpoint inhibitors include nivolumab (anti-PD-1 antibody); and examples of non-specific immunostimulants include OK-432, dried BCG, trametes versicolor-polysaccharide preparation, lentinan, ubenimex, etc. In addition, aceglatone, porfimer sodium, talaporfin sodium, ethanol, arsenic trioxide, and the like can also be used.

When hematopoietic organ tumors are targeted, the molecularly targeted drug is preferably imatinib mesilate, nilotinib hydrochloride hydrate, or dasatinib hydrate. When solid tumors are targeted, the molecularly targeted drug is preferably gefitinib, erlotinib hydrochloride, or lapatinib tosilate hydrate, and particularly preferably imatinib mesilate.

Examples of ICIs other than the above include anti-CTLA-4 antibodies (e.g., ipilimumab and tremelimumab), anti-PD-1 antibodies (e.g., pembrolizumab, PDR001 (codename), and cemiplimab), anti-PD-L1 antibodies (e.g., avelumab, atezolizumab, durvalumab, MPDL3280A, and MEDI4736), etc., all of which target CTLA-4, PD-1, or PD-L1 as an immune checkpoint molecule. These ICIs are drugs currently in development for the treatment of tumors.

In an embodiment in which the other antitumor agents etc. and the PAI-1 inhibitor are administered simultaneously, for example, the other antitumor agents etc. and the PAI-1 inhibitor are formulated into a single pharmaceutical composition (combination drug). Specifically, the pharmaceutical composition is a combination drug in a desired dosage form containing the other antitumor agents etc. and the PAI-1 inhibitor, and optionally containing a pharmacologically acceptable carrier or additive. The PAI-1 inhibitor is preferably, but is not limited to, at least one member selected from compound group 1 represented by Formula (I) described in section I. The carrier or additive used herein may be one that does not impair the effects of the tumor chemotherapy agent of the present invention. The details thereof will be described in the following section IV. The form of the combination drug can be suitably determined depending on the administration route (dosage form) thereof; the form is preferably suitable for oral administration.

The administration route (administration method) is not particularly limited. Examples thereof include oral administration, and parenteral administration such as intravenous administration, intramuscular administration, subcutaneous administration, transmucosal administration, transdermal administration, and intrarectal administration, depending on the form of the preparation. Preferable are oral administration and intravenous administration, and more preferable is oral administration. The form of the combination drug depending on each administration route (administration method) and the method for producing the same will be described in the following "IV. Pharmaceutical composition" section.

In another embodiment in which the other antitumor agents etc. and the PAI-1 inhibitor are simultaneously administered, for example, the other antitumor agents etc. and the PAI-1 inhibitor in different packaging forms are mixed immediately before application to a tumor patient. Specific examples thereof include a tumor chemotherapy agent that is used such that the PAI-1 inhibitor is added and mixed with an antitumor agent etc. in the form of a liquid (e.g., infusion or oral liquid) immediately before administration to a tumor patient (before use).

In this case, the combination ratio of the other antitumor agents etc. and the PAI-1 inhibitor is not limited, as long as the effects of the present invention are obtained. The ratio can be suitably determined depending on the type and location of tumor to be treated, the stage (severity and progression) of the tumor, the pathological conditions, age, sex, and body weight of the tumor patient, the type of antitumor agent etc. to be used in combination, etc.

Specifically, for example, when the tumor chemotherapy agent is an orally administered agent, the dosage thereof in terms of the amount of the PAI-1 inhibitor, which is an active ingredient, can be suitably determined within the range of 0.01 to 300 mg/kg/day, preferably 0.03 to 30 mg/kg/day, and more preferably 0.1 to 10 mg/kg/day. This range includes 0.5 to 5 mg/kg/day.

When the PAI-1 inhibitor and the other antitumor agents etc. are combined, the dosage of the other antitumor agents etc. to be combined can also be suitably determined within the range of 0.1 to 5,000 mg/kg/day, 0 to 5,000 mg/kg/time, 0.1 to 5,000 mg/kg/time, 0.1 to 5,000 mg/m²/time, or 100,000 to 5 million JRU/time. The dosage can be suitably determined within the range of 0.2 to 5 times larger than the recommended dosage of the agent. The dosage is more preferably 0.5 to 2 times larger than the recommended dosage of the agent.

As for the dose frequency, administration can be continued for 1 to 8 days, and drug holidays of 5 days to 9 weeks can be provided.

When the tumor chemotherapy agent of the present invention is an intravenously administered agent, it can be administered in an amount of 0.03 to 300 mg/kg per day so that the effective blood level of the PAI-1 inhibitor is 0.2 to 50 µg/mL, and preferably 0.5 to 20 µg/mL. When the other antitumor agents etc. are combined, the dosage thereof can be suitably determined within the range of 10 to 100%, preferably 10 to 95%, of the general dosage.

Furthermore, when the tumor chemotherapy agent of the present invention is administered, the PAI-1 inhibitor can be singly administered after the antitumor treatment according to the above method, thereby inhibiting exacerbation (including recurrence and metastasis) of the tumor.

In this case, the dosage of the PAI-1 inhibitor can be suitably determined within the range of 0.003 to 3,000 mg/kg/day. Moreover, in this case, the administration is preferably continued for at least 1 year or more, more preferably 5 years or more, and even more preferably for the entire life.

In an embodiment in which the other antitumor agents etc. and the PAI-1 inhibitor are separately administered, for example, the other antitumor agents etc. and the PAI-1 inhibitor in different packaging forms are administered to a tumor patient simultaneously or at different times. In the embodiment of simultaneous administration, for example, the other antitumor agents etc. and the PAI-1 inhibitor both in oral dosage forms are orally administered simultaneously; or when one of them is in an oral dosage form, and the other is in a parenteral dosage form, both can be administered simultaneously or in parallel. In the embodiment of administration at different times, for example, the other antitumor agents etc. and the PAI-1 inhibitor both in oral dosage forms are orally administered at different times; or when one of them is in an oral dosage form, and the other is in a parenteral dosage form, both can be administered at different times. In the latter case, the order of administration is either of the following: the PAI-1 inhibitor is administered before the administration of the other antitumor agents etc., or the PAI-1 inhibitor is administered after the administration of the other antitumor agents etc. The PAI-1 inhibitor and the other antitumor agents etc. used in this case are provided in the form of a desired pharmaceutical composition, together with a pharmacologically acceptable carrier or additive, depending on the administration route (dosage form). The type of pharmacologically acceptable carrier or additive, and the method for producing the same will be described in the following section IV.

The tumor chemotherapy agent of the present invention obtained by combining the other antitumor agents etc. with the PD-L1 expression inhibitor, immunostimulator, ICI, tumor cell exacerbation inhibitor, or immunotherapy enhancer of the present invention is capable of supplementing and enhancing the action of the other antitumor agents etc. used in combination, based on the mechanism of action of the PAI-1 inhibitor for use in the various applications mentioned above. As a result, it is possible to achieve more effective antitumor treatment and provide an excellent or improved prognosis of antitumor treatment by inhibiting the tumor cell proliferation, infiltration, metastasis, and/or tumor recurrence after antitumor treatment (chemotherapy, radiotherapy, and surgical treatment such as ablative operation). Therefore, the tumor chemotherapy agent of the present invention can be effectively applied especially to patients who have tumor cells with a high level of PD-L1 expression, and preferably patients who have tumors with a high grade of malignancy or having progressive tumors.

The "prognosis of antitumor treatment" mentioned herein can be evaluated according to, for example, the tumor recurrence rate, metastasis rate, five-year survival rate, or ten-year survival rate of the tumor patient after antitumor treatment.

### IV. Pharmaceutical Composition

The PD-L1 expression inhibitor, immunostimulator, ICI, tumor cell exacerbation inhibitor, enhancer of immunotherapy for tumors, and tumor chemotherapy agent of the present invention described above are prepared as pharmaceutical compositions in predetermined forms (dosage forms), and administered to target patients. Therefore, the above preparations are collectively referred to as the "pharmaceutical composition of the present invention."

The pharmaceutical composition of the present invention may consist of 100 wt.% of the PAI-1 inhibitor, which is its active ingredient; however, the pharmaceutical composition of the present invention usually comprises a pharmacologically acceptable carrier or additive, in addition to the PAI-1 inhibitor. In the latter case, the ratio of the PAI-1 inhibitor in the pharmaceutical composition is not limited; however, the ratio can be generally selected from the range of 5 to 95 wt.%, and preferably 30 to 80 wt.%.

The pharmaceutical composition of the present invention can be administered orally or parenterally, such as intravenously, intramuscularly, subcutaneously, transmucosally, transdermally, and intrarectally. Among these, preferable are oral administration and intravenous administration, and more preferable is oral administration. The pharmaceutical composition of the present invention can be provided in various forms of preparations (dosage forms) depending on the above-mentioned administration manners.

Each dosage form is described below; however, the dosage forms employed in the present invention are not limited thereto. Any dosage forms that are usually used in the field of pharmaceutical preparations can be employed.

In the case of oral administration, the dosage form of the pharmaceutical composition of the present invention is suitably selected from powders, granules, capsules, pills, tablets, elixirs, suspensions, emulsions, and syrups. Such preparations can be imparted with sustained-release properties, stabilization, easy degradation, difficult degradation, enteric properties, easy-adsorption properties, etc.

In the case of intravenous administration, intramuscular administration, or subcutaneous administration, the dosage form can be suitably selected from injections or drops (including dried products that are prepared upon use), and the like.

In the case of transmucosal administration, transdermal administration, or intrarectal administration, the dosage form can be suitably selected from masticatories, sublingual agents, buccal tablets, troches, ointments, patch agents, liquid agents, etc., according to the applied portion. Such preparations can be imparted with sustained-release properties, stabilization, easy degradation, difficult degradation, easy-adsorption properties, etc.

The pharmaceutical composition of the present invention can contain a pharmaceutically acceptable carrier and additive according to the dosage form (for oral administration or various parenteral administrations). Examples of pharmaceutically acceptable carriers and additives include solvents, excipients, coating agents, bases, binders, lubricants, disintegrators, solubilizers, suspending agents, thickening agents, emulsifiers, stabilizers, buffers, isotonizing agents, soothing agents, preservatives, corrigents, flavors, and coloring agents. Specific examples of pharmaceutically acceptable carriers and additives are mentioned below; however, the present invention is not limited thereto.

Examples of solvents include purified water, sterile purified water, water for injection, physiologic saline, peanut oil, ethanol, glycerol, etc. Examples of excipients include starches (e.g., potato starch, wheat starch, and corn starch), lactose, dextrose, saccharose, crystalline cellulose, calcium sulfate, calcium carbonate, sodium hydrogencarbonate, sodium chloride, talc, titanium oxide, trehalose, xylitol, etc.

Examples of binders include starch and starch derivatives, cellulose and cellulose derivatives (e.g., methylcellulose, ethylcellulose, hydroxypropylcellulose, and carboxymethylcellulose), natural high-molecular-weight compounds, such as gelatin, sodium arginine, tragacanth, gum arabic, etc., synthetic high-molecular-weight compounds, such as polyvinyl pyrrolidone, polyvinyl alcohol, etc., dextrin, hydroxypropyl starch, and the like.

Examples of lubricants include light anhydrous silicic acid, stearin acid and salts thereof (e.g., magnesium stearate), talc, waxes, wheat starch, macrogol, hydrogenated vegetable oil, sucrose fatty acid ester, polyethylene glycol, silicone oil, etc.

Examples of disintegrators include starch and starch derivatives, agar, gelatin powder, sodium hydrogencarbonate, calcium carbonate, cellulose and cellulose derivatives, hydroxypropyl starch, carboxymethylcellulose, salts thereof, and bridging materials thereof, low-substituted hydroxypropylcellulose, etc.

Examples of solubilizers include cyclodextrin, ethanol, propylene glycol, polyethylene glycol, etc. Examples of suspending agents include sodium carboxymethylcellulose, polyvinylpyrrolidone, gum arabic, tragacanth, sodium arginine, aluminum monostearate, citric acid, various surfactants, etc.

Examples of thickening agents include sodium carboxymethylcellulose, polyvinylpyrrolidone, methylcellulose, hydroxypropyl methylcellulose, polyvinyl alcohol, tragacanth, gum arabic, sodium arginine, etc.

Examples of emulsifiers include gum arabic, cholesterol, tragacanth, methylcellulose, lecithin, various surfactants (e.g., polyoxyl 40 stearate, sorbitan sesquioleate, polysorbate 80, and sodium lauryl sulfate), etc.

Examples of stabilizers include tocopherol, chelating agents (e.g., EDTA and thioglycolic acid), inert gases (e.g., nitrogen and carbon dioxide), reducing substances (e.g., sodium hydrogen sulfite, sodium thiosulfate, ascorbic acid, and rongalite), etc.

Examples of buffers include sodium hydrogenphosphate, sodium acetate, sodium citrate, boric acid, etc.

Examples of isotonizing agents include sodium chloride, glucose, etc. Examples of soothing agents include local anesthetics (e.g., procaine hydrochloride and lidocaine), benzyl alcohol, glucose, sorbitol, amino acid, etc.

Examples of corrigents include saccharose, saccharin, *Glycyrrhiza* extract, sorbitol, xylitol, glycerol, etc. Examples of flavoring agents include orange peel tincture, rose oil, etc. Examples of coloring agents include water-soluble food colors, lake pigment, etc.

Examples of preservatives include benzoic acid and salts thereof, p-hydroxybenzoate esters, chlorobutanol, invert soap, benzyl alcohol, phenol, thimerosal, dehydroacetic acid, boric acid, etc.

Examples of coating agents include saccharose, hydroxypropylcellulose (HPC), shellac, gelatin, glycerol, sorbitol, hydroxypropyl methylcellulose (HPMC), ethylcellulose, polyvinyl pyrrolidone (PVP), hydroxypropylmethylcellulose phthalate (HPMCP), cellulose acetate phthalate (CAP), methyl methacrylate-methacrylic acid copolymer, polymers described above, etc.

Examples of bases include Vaseline, liquid paraffin, carnauba wax, beef tallow, hardened oil, paraffin, yellow beeswax, vegetable oil, macrogol, macrogol fatty acid ester, stearic acid, sodium carboxymethylcellulose, bentonite, cacao butter, Witepsol, gelatin, stearyl-alcohol, hydrous lanolin, cetanol, light liquid paraffin, hydrophilic petrolatum, simple ointment, white ointment, hydrophilic ointment, macrogol ointment, hard fat, oil-in-water emulsion bases, water-in-oil emulsion bases, etc.

Known drug delivery systems (DDS) can be applied for the dosage forms given above. The term "DDS preparation" as used in the present specification refers to slow-release preparations, locally applied preparations (troches, buccal tablets, sublingual tablets, etc.), drug control-release preparations, enteric coated preparations, gastric soluble preparations, etc., that are all prepared in the best form considering the administration route, bioavailability, side effects, etc.

### Examples

Below, the present invention is described in more detail with reference to Reference Experimental Examples and Experimental Examples. However, the present invention is not limited to these examples.

### Reference Experimental Examples

### Reference Experimental Example 1: Measurement of PAI-1 Inhibitory Activity of Compound Group 1

The compounds (compound examples 1 to 7 and 9 to 14) shown in Table 1 that fall within the scope of compound group 1 represented by Formula (I) and existing compounds 1 and 2 known as a PAI-inhibitor (see Table 1) were measured and evaluated for inhibitory effect on human PAI-1 (manufactured by Molecular Innovations, Inc. (USA); the same applies hereinafter).

Specifically, human-derived PAI-1 was added to a 0.1% Tween 80-containing 100 mM Tris-HCl (pH 8) solution containing each of the above compounds in a given concentration (0.29 mM or 0.12 mM), and the mixture was incubated at 37°C for 15 minutes. Subsequently, human-derived tissue plasminogen activator (t-PA) (manufactured by American Diagnostica, Inc. (USA); the same applies hereinafter) adjusted to 0.35 pmol/µL was added thereto, and the mixture was further incubated at 37°C for 15 minutes. Then, 1.25 mM of S-2288 synthetic substrate (manufactured by Chromogenix, (Italy); the same applies hereinafter), which was a chromogenic substrate, was added. The final mixture contained 100 mM Tris-HCl (pH 8), 30 mM NaCl, 1% DMSO, 0.1% Tween 80, 67 nM PAI-1, 9.8 nMt-PA, 1 mM S-2288 synthetic substrate, and each compound (50 µM or 20 µM).

Free p-nitroaniline removed from the chromogenic substrate (S-2288) by t-PA action was measured using a spectrophotometer at an absorbance of 405 nm at 5-minute intervals for 30 minutes. A system that did not contain each of compound examples 1 to 7 and 9 to 14 was similarly evaluated, and the PAI-1 activity of the system after 30 minutes was taken as 100% to evaluate the PAI-1 activity of a system to which a test compound was added. Table 1 shows the results.

### Reference Experimental Example 2: Measurement of PAI-1 Inhibitory Activity of Compound Group 1

The compounds shown in Table 2 that fall within the scope of compound group 1 represented by Formula (I) and the existing compounds 1 to 4 known as a PAI-1 inhibitor were used as a test compound and evaluated for inhibitory effect on human PAI-1 (manufactured by Molecular Innovations, Inc. (USA) ; the same applies hereinafter).

Specifically, human PAI-1 was added to a 0.1% PEG-6000and 0.2 mM CHAPS-containing 50 mM tris-HCL (pH 8) solution containing each of the above compounds in a given concentration (62.5 µM or 15.6 µM), and the mixture was incubated at 37°C for 15 minutes. Subsequently, human-derived tissue plasminogen activator (t-PA) (manufactured by American Diagnostica, Inc. (USA); the same applies hereinafter) adjusted to 0.05 pmol/pL was added thereto, and the mixture was further incubated at 37°C for 60 minutes. Then, 0.25 mM of Spectrozyme t-PA synthetic substrate (manufactured by American Diagnostica, Inc. (USA); the same applies hereinafter), which is a chromogenic substrate, was added. The final mixture contained 50 mM Tris-HCl (pH 8), 150 mM NaCl, 1% DMSO, 0.1% PEG-6000, 0.2 mM CHAPS, 5nM PAI-1, 2nM t-PA, 0.2 mM Spectrozyme t-PA synthetic substrate, and each compound (10 µM or 2.5 pM).

Free p-nitroaniline removed from the chromogenic substrate (Spectrozyme t-PA) by t-PA action was measured using a spectrophotometer at an absorbance of 405 nm at 20-minute intervals, for 120 minutes. A system that did not contain the test compounds was similarly evaluated, and the PAI-1 activity of the system after 120 minutes was taken as 100% to evaluate the PAI-1 activity of a system to which a test compound was added. Table 2 shows the results.

### Experimental Examples

As described below, experiments were conducted using the following test compound (Example 68) as a test compound. However, this test compound is merely one example of the PAI-1 inhibitor as subject matter of the present invention, and it is clear from the experimental results described below that similar results are also obtained with the use of compounds having a PAI-1 inhibitory effect (PAI-1 inhibitor).

### Test Compound

### Sodium 5-chloro-2-({[3-(quinolin-8-yl)phenyl]carbonyl}amino)benzoate

This compound belongs to compound group 1 represented by Formula (I). The compound was produced according to the disclosure of WO 2010/113022. The bioisosteres of the compound can be produced following (or in accordance with) the disclosure of WO 2010/113022. The compound has been confirmed to have no safety issues when used by oral administration in non-clinical and clinical studies in mammals, especially humans (JP2020-088416A).

Laboratory animals were raised at the animal facility of Tokai University School of Medicine. Animal experiments were conducted with the approval of the Animal Care Committee of Tokai University and in accordance with the ethical guidelines of the committee.

In the following experiments, flow cytometry analysis was performed with LSRFortessa (BD Bioscience). Each analysis was performed on 1,000,000 cells, and FlowJo was used for analysis.

### Experimental Example 1

In order to analyze the characteristics of cancer cells highly expressing PAI-1, a mouse leukemia cell line (obtained by transfecting 32D cells, i.e., a myelomonocytic progenitor cell line, with BCR-ABL; referred to below as "32Dp210") was transfected with PAI-1 to produce cells overexpressing PAI-1 (32d PAI-1 OE). Next, in order to analyze the expression level of PD-L1, which is an ICM, in the cells, the cells were stained with an anti-PD-L1 antibody (clone name: 10F.G92) labeled with Allophycocyanin (APC) as a fluorescent dye, and subjected to flow cytometry. As a comparative control experiment, a 32Dp210 cell line without transfection of PAI-1 (wild-type cells: 32D WT) was analyzed in the same manner for the PD-L1 expression level.

Fig. 1 shows the results from flow cytometer. The results shown in Fig. 1 clarified that the 32Dp210 overexpressing PAI-1 (32D PAI-1 OE) had a higher PD-L1 expression level than that of the control, i.e., untreated wild-type cells (32D WT).

Tumor cells highly expressing PAI-1 have a high grade of malignancy. It was thus suggested that tumor cells highly expressing PAI-1 highly express PD-L1 to thus escape from the surveillance mechanism (immune surveillance mechanism) by immune cells, whereby metastasis etc. become likely to occur, increasing the potential of malignancy.

### Experimental Example 2

PAI-1 is a protein that functions intracellularly and extracellularly. When secreted extracellularly, PAI-1 induces a variety of responses in itself and in surrounding cell populations. Therefore, the effect of PAI-1 secreted from tumor cells on PD-L1 expression was studied.

Specifically, artificially produced recombinant PAI-1 (referred to below as "rPAI-1"; 40 nM) or a culture supernatant of the cells overexpressing PAI-1 (32d PAI-1 OE) prepared in Experimental Example 1 (PAI-1 OE sup; containing a high concentration of PAI-1) was added to 5 × 10⁴ cells of a mouse leukemia cell line (32Dp210), and the cells were cultured on a 96-well plate at 37°C, 5% CO₂, for 2 days. Next, the PD-L1 expression level was determined by flow cytometry in the same manner as in Experimental Example 1. As a comparative control experiment, the PD-L1 expression level in untreated mouse leukemia cells (control) was measured in the same manner.

Fig. 2 shows the results from flow cytometry. The results shown in Fig. 2 clarified that the cells to which PAI-1 was added (+r-PAI-1, +PAI-1 OE sup) were both induced to highly express PD-L1, compared with untreated cells (control).

These results clarified that PAI-1 secreted from tumor cells induces PD-L1 expression in tumor cells. The results also suggested the possibility that PAI-1 secreted from tumor cells also induces PD-L1 expression in cell populations constituting the tumor-surrounding environment (tumor-associated macrophages (TAMs) and cancer-associated fibroblasts (CAFs)) to evade attack from immune cells.

### Experimental Example 3

Based on the results of Experimental Example 2, a test compound having a PAI-1 inhibitory effect (referred to below as "the PAI-1 inhibitor") was added to a mouse leukemia cell line (32Dp210) to a concentration of 50 µM, together with rPAI-1 (40 nM), and the cells were cultured on a 96-well plate at 37°C, 5% CO₂, for 2 days. Next, the PD-L1 expression level was determined by flow cytometry in the same manner as in Experimental Example 1.

Fig. 3(A) shows the results. As shown in Fig. 3(A), PD-L1 expression induction by the addition of rPAI-1 observed in Experimental Example 2 was completely suppressed by the addition of the PAI-1 inhibitor. These results suggest that the PAI-1 inhibitor provides restoration from immune system dysfunction (immune depression) caused by PD-L1 by suppressing PD-L1 expression induction by PAI-1, thereby exerting an immunostimulatory effect.

Likewise, a mouse colorectal cancer cell line (MC38) and a mouse malignant melanoma cell line (B16F10) were also tested in the same manner, instead of the mouse leukemia cell line (32Dp210). As shown in Figs. 3(B) and 3(C), the colorectal cancer cells and the malignant melanoma cells both had the same results as those of the leukemia cells.

Based on the above, malignant alteration due to PD-L1 induction by PAI-1 is highly possibly a principle that is observed in common with many tumor types, regardless of whether hematopoietic organ tumors or solid tumors. The PAI-1 inhibitor is expected to inhibit PD-L1 induction caused by PAI-1, which occurs in such a wide range of tumor types, suppress immune depression caused by the PD-L1 induction, and suppress exacerbation of tumors caused by the PD-L1 induction (suppress exacerbation of tumors by immune stimulation).

### Experimental Example 4

The culture supernatant of the cells overexpressing PAI-1 (32D PAI-1 OE) prepared in Experimental Example 1 (PAI-1 OE sup) was added to a mouse leukemia cell line (32Dp210). In addition, those obtained by adding the PAI-1 inhibitor to a concentration of 50 µM, together with rPAI-1 (40 nM or 100 nM), were also prepared. Then, culturing was performed on a 96-well plate at 37°C, 5% CO₂, overnight. The cells were collected, RNA was extracted using ISOGEN2 (Nippon Gene Co., Ltd.), and 100 ng of RNA was subjected to a reverse transcription reaction using a PrimeScript RT-PCR kit (Takara Bio Inc.) to obtain cDNA. The PD-L1 gene expression level was monitored by real-time PCR using TaqMan Fast Advanced Master Mix (Thermo Fisher Scientific). PD-L1 (Mm00452054_m1, Thermo Fisher Scientific) was used as the primer. To standardize the RNA amount, the expression level of 18S rRNA (Hs99999901_s1, Thermo Fisher Scientific) was also measured simultaneously. The PD-L1 gene expression level was determined using a comparative Ct method, which was shown in the graph as a ratio relative to the control group.

Fig. 4 shows the results.

As shown in Figs. 4(A) and 4(B), the addition of the cells overexpressing the PAI-1 gene (PAI-1 OE) or rPAI-1 (40 nM or 100 nM) not only resulted in the expression of PD-L1 protein on the surface of the mouse leukemia cells as confirmed in Experimental Examples 1 and 2, but also resulted in an increase in the expression of PD-L1 mRNA. The increase in mRNA expression was suppressed by the addition of the PAI-1 inhibitor (50 µM), as shown in Fig. 4(B).

These results indicate that it is highly possible that not only the low-molecular-weight compound used in this experiment, but also neutralizing antibodies and nucleic acid drugs would also effectively achieve PAI-1 inhibition for PA-L1-dependent tumors in antitumor therapy.

### Experimental Example 5

The effectiveness of the PAI-1 inhibitor in *vivo* was studied.

Specifically, 5 × 10⁵ cells of a mouse leukemia cell line (32Dp210) were transplanted into mice (C3H/HEJ), and engraftment was confirmed one week later. Subsequently, the PAI-1 inhibitor was orally administered once daily at 10 mg/kg for a total of 7 days. On the next day of the final administration, the peripheral blood or the spleen was collected from the mice. The spleen was mechanically dispersed by grinding through nylon mesh. The peripheral blood and spleen cells were independently hemolyzed with an ammonium chloride solution and then stained with an anti-PD-L1 antibody (clone name: 10F.G92) labeled with Allophycocyanin (APC) to determine the PD-L1 expression level in the leukemic cells with a flow cytometer. As a comparative control experiment, instead of the PAI-1 inhibitor, only physiological saline (vehicle) was administered to the mice transplanted with the mouse leukemia cell line, and the PD-L1 expression level in the leukemic cells was determined with a flow cytometer in the same manner as described above.

Fig. 5 shows the results of the peripheral blood. (The spleen also showed the same results.)

Fig. 5 shows that that the mice administered with the PAI-1 inhibitor had a lower PD-L1 expression level in leukemia cells than the mice administered with physiological saline (vehicle) alone. These results suggest that the immunostimulatory effect of the PAI-1 inhibitor shown in the results of Experimental Examples 3 and 4 would be exerted not only *in vitro,* but also in *vivo* under cancer-bearing conditions that reflect the clinical conditions of tumor patients and are closer to physiological conditions.

### Experimental Example 6

The effect of PAI-1 on TAMs (M2 macrophages) was analyzed.

Specifically, 5 × 10⁶ mouse spleen cells were cultured on a 24-well plate at 37°C, 5% CO₂, overnight. After floating cells were removed, rPAI-1 (100 nM) was added, and culturing was performed at 37°C, 5% CO₂, for 4 days. After culture, adherent cells were collected by trypsin treatment and stained with an anti-CD11b antibody and an anti-CD206 antibody, in addition to an anti-PD-L1 antibody. Then, gating was performed on CD11b and CD206 double-positive (CD11b⁺CD206⁺) M2 macrophages, and the PD-L1 expression level thereof was determined with a flow cytometer. As a comparative control experiment, the PD-L1 expression level in untreated mouse spleen cells (control) was determined in the same manner. Further, the PAI-1 inhibitor (50 µM) was added to the same mouse spleen cells, together with rPAI-1 (100 nM), followed by culture under the same conditions as described above. Then, adherent cells were collected and the PD-L1 expression level was determined with a flow cytometer in the same manner.

Fig. 6 shows the results.

The results shown in Fig. 6 clarified that PD-L1 expression was highly induced in M2 macrophages to which rPAI-1 was added, compared with untreated cells (control). Enhancement of PD-L1 expression by PAI-1 was completely suppressed with the addition of the PAI-1 inhibitor to the culture system to which rPAI-1 was added. These results confirmed that tumor cells secrete PAI-1 to induce PD-L1 expression also in M2 macrophages (TAMs) and other immunosuppressive cells constituting the tumor-surrounding environment, to thus evade attack from the immune system. The results also confirmed that the use of the PAI-1 inhibitor can inhibit the immunosuppressive effect.

### Experimental Example 7

The following experiments were performed to clarify the association between PAI-1 and PD-L1 in M2 macrophages (TAMs) and the effect of the PAI-1 inhibitor on them.

### (1) Association between PAI-1 and PD-L1 in M2 macrophages (TAMs)

In regards to mouse spleen cells, an anti-CD11b antibody and an anti-CD206 antibody were used to stain M2 macrophages (CD11b⁺CD206⁺), which were further stained with an antibody against PD-L1 or PAI-1, followed by analysis with a flow cytometer in the same manner as in Experimental Example 6.

Fig. 7 shows the results.

The results shown in Figs. 7(B) and 7(C) clarified that the M2 macrophages (CD11b⁺CD206⁺) had a higher expression level of PAI-1 (Fig. 7(B)), compared with other macrophages (CD11b⁺CD206⁻), and in correlation with this, also had a higher expression level of PD-L1 (Fig. 7(C)).

### (2) Effect of PAI-1 Inhibitor

Mice (C57BL6) were orally administered with the PAI-1 inhibitor once daily at 10 mg/kg for a total of 7 days. On the next day of the final administration, the peripheral blood or the spleen was collected from the mice. The spleen was mechanically dispersed through nylon mesh. The peripheral blood and spleen cells were independently hemolyzed with an ammonium chloride solution, and the PD-L1 expression level in M2 macrophages (CD11b⁺CD206⁺) was determined with a flow cytometer. As a comparative control experiment, the peripheral blood or the spleen was collected in the same manner from a mouse group to which only physiological saline (vehicle) was administered to determine the PD-L1 expression level in M2 macrophages (CD11b⁺CD206⁺) .

Fig. 7 shows the results of the peripheral blood (the spleen also showed the same results).

As shown in Fig. 7(D), the mouse group to which the PAI-1 inhibitor was orally administered resulted in a reduction in the PD-L1 expression level in M2 macrophages (CD11b⁺CD206⁺) . The results clarified that immunosuppressive M2 macrophages express a high level of PAI-1 and PD-L1, and that the PD-L1 expression is reduced by the PAI-1 inhibitor. In other words, it was clarified that PD-L1 expression is regulated by PAI-1, and that PD-L1 expression can be reduced by suppressing the PAI-1 activity with the PAI-1 inhibitor.

### Experimental Example 8

Whether the same phenomenon would be reproduced in human cells in the same manner as in the mouse cells as demonstrated in Experimental Examples 1 to 7 was verified.

Specifically, cells overexpressing the PAI-1 gene (PAI-1 OE) were produced using human cells, i.e., 293T (immortalized fetal renal cells), ES2 (ovarian clear cell adenocarcinoma cells), MOLM14 (acute myelogenous leukemia cells), and K562 (chronic myelogenous leukemia cells). Alternatively, a culture supernatant of the cells (PAI-1 OE) produced as described above (PAI-1 OE sup: containing a high concentration of PAI-1) was added to the above human cells. Then, culturing was performed on a 96-well plate at 37°C, 5% CO₂, for 2 days. After culture, the PD-L1 expression level in each of the cells was determined with a flow cytometer. As a comparative control experiment, untreated wild-type cells (WT) in terms of each of the cells were cultured in the same manner, and the PD-L1 expression level after culture was determined with a flow cytometer.

Fig. 8 shows the results.

Fig. 8 confirmed that the PD-L1 expression level increased in all of the human cells, in terms of both the cells overexpressing the PAI-1 gene (PAI-1 OE) and the cells to which PAI-1 was added (+PAI-1 OE sup), compared with the control, i.e., untreated wild-type cells (WT). These results indicate that the induction of PD-L1 expression by PAI-1 is a phenomenon observed not only in mice but also in humans, and that the PAI-1 inhibitor can block the immune checkpoint mechanism of PD-L1 also in human cells by inhibiting PAI-1 and is thus believed to be useful as an immunostimulator in a wide variety of human tumor types, irrespective of whether they are liquid or solid tumors.

### Experimental Example 9

The efficacy of the PAI-1 inhibitor against human cancer cells (ovarian cancer cells) was studied *in vivo.* Specifically, 5 × 10⁵ cells of a human ovarian clear cell adenocarcinoma cell line (ES2) were transplanted subcutaneously in nude mice. One week later, engraftment of more than 5 mm of tumor mass in the major axis was confirmed. Subsequently, the PAI-1 inhibitor was orally administered once daily at 10 mg/kg for a total of 7 days. On the next day of the final administration, the tumor mass was collected from the mice, chopped with scissors, suspended in a 0.025% collagenase solution (FUJIFILM Wako Pure Chemical Corporation), and treated at 37°C for 2 hours to break cell-cell bonds. The cells obtained by enzymatic treatment were stained with an anti-PD-L1 antibody (clone name: 10F.G92) labelled with Allophycocyanin (APC), and the PD-L1 expression level in the tumor cells was determined with a flow cytometer. As a comparative control experiment, cells were collected also from the tumor mass in the same manner in terms of a mouse group to which only physiological saline (vehicle) was administered instead of the PAI-1 inhibitor, and the PD-L1 expression level in the tumor cells was determined with a flow cytometer.

Fig. 9 shows the results.

As shown in Fig. 9, the PD-L1 expression level in the tumor cells was reduced in the group to which the PAI-1 inhibitor was orally administered (+PAI-1 inhibitor), compared with the group to which only physiological saline (vehicle) was administered. The results clarified that the PAI-1 inhibitor is effective against human tumor cells *in vivo* as well.

### Experimental Example 10

The antitumor effect of the PAI-1 inhibitor was studied.

Specifically, 5 × 10⁵ cells of a mouse leukemia cell line (32Dp210) were transplanted subcutaneously in mice (C3H/HEJ), and one week later, engraftment of more than 5 mm of a tumor mass in the major axis was confirmed. Subsequently, the PAI-1 inhibitor was orally administered once daily at 10 mg/kg for a total of 7 days. On the next day of the final administration, the size of the tumor mass (tumor diameter, mm³) was measured. As a comparative control experiment, the size of the tumor mass was measured in the same manner in terms of a mouse group to which only physiological saline (vehicle) was orally administered instead of the PAI-1 inhibitor.

Fig. 10 shows the results.

According to Fig. 10, the mouse groups (n=2) to which only physiological saline (vehicle) was administered (codes 1 and 2) showed an increase in the tumor diameter, whereas the mouse groups (n=2) to which the PAI-1 inhibitor was administered (codes 3 and 4) showed a loss of tumor mass. These results indicate that the PAI-1 inhibitor itself exerts an antitumor effect *in vivo.*

According to the experimental results above, the PAI-1 inhibitor acts to inhibit the induction of PD-L1 expression caused by PAI-1 by inhibiting PAI-1. Thus, the PAI-1 inhibitor as a single agent is useful as a PD-L1 expression inhibitor, immunostimulator, immune checkpoint inhibitor (ICI) targeting PD-L1, inhibitor for exacerbation of tumor cells caused by PD-L1, or enhancer of immunotherapy for tumors. The results also indicated the potentiality of the PAI-1 inhibitor as a single agent or in combination with an antitumor agent, other immunostimulators, or other ICIs, to exhibit high therapeutic efficacy in mammals suffering from tumors, especially in mammals, including humans, suffering from highly malignant, advanced cancers caused by PAI-1.

### Experimental Example 11

The same test was performed using a mouse colorectal cancer cell line (MC38) instead of the mouse leukemia cell line used in Experimental Example 10, and the antitumor effect of the PAI-1 inhibitor was studied.

Mice (C57BL6) were divided into 1) an untreated group (vehicle) (n=15), 2) a single-administration group of anti-PD1 antibody (anti-PD-1 Ab), 3) a single-administration group of PAI-1 inhibitor (PAI-1 inhibitor), and 4) a combination group of anti-PD1 antibody and PAI-1 inhibitor (anti-PD-1 Ab+PAI-1 inhibitor) (n=18 for 2) to 4)). Then, 5 × 10⁵ cells of the mouse colorectal cancer cell line (MC38) were transplanted subcutaneously into each mouse. One week after transplantation, engraftment of more than 5 mm of a tumor mass in the major axis was confirmed. Subsequently, the PAI-1 inhibitor (10 mg/kg) was orally administered once daily to groups 3) and 4) above for a total of 14 consecutive days. For groups 2) and 4), 15 µg of anti-PD1 antibody was intraperitoneally administered every 3 days (3 times in total) from the same day as the administration day of the PAI-1 inhibitor. For the untreated group, physiological saline was orally administered. The size of the tumor mass was measured 2 weeks (14 days) and 3 weeks (21 days) after the transplantation (0 days).

Fig. 11 shows the results.

As shown in Fig. 11, 1) the untreated group (-●-) showed an increase in the tumor diameter, whereas 2) the single-administration group of anti-PD-1 antibody (-▲-) and 3) the single-administration group of PAI-1 inhibitor (-■-) showed tumor regression. Further, 4) the combination group of anti-PD-1 antibody and PAI-1 inhibitor (- 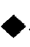 -) showed complete disappearance of the tumor mass. These results indicate that the PAI-1 inhibitor and the anti-PD-1 antibody each exhibit an antitumor effect independently, and that when used in combination, their antitumor effects are enhanced.

### Experimental Example 12

In Experimental Example 11, the single-administration of PAI-1 inhibitor was confirmed to exhibit an antitumor effect. This is presumably because tumor immunity was activated by the PAI-1 inhibitor.

To confirm this point, the following experiment was performed. Specifically, 5 × 10⁵ cells of a mouse colorectal cancer cell line (MC38) were subcutaneously transplanted into immunodeficient mice (Rag2/IL-2R-KO) and wild-type mice (WT, for control). The immunodeficient mice lacked the RAG2 gene and the IL-2 receptor γ chain gene, and immune cells (T cells, B cells, NK cells) were not congenitally present in the body. One week later, engraftment of more than 5 mm of a tumor mass in the major axis was confirmed. Subsequently, the PAI-1 inhibitor (10 mg/kg) or physiological saline (vehicle) was orally administered to each mouse once daily for a total of 14 consecutive days.

Fig. 12 shows the results. As shown in Fig. 12, the tumor regression effect of the PAI-1 inhibitor, which was observed in the wild-type mice, was not observed in the immunodeficient mice. These results clarified that the PAI-1 inhibitor causes tumor regression by activating immunity.

### Experimental Example 13

1) The untreated group and 3) the single-administration group of PAI-1 inhibitor (cancer-bearing mice) of Experimental Example 11, after tumor engraftment, were orally administered with physiological saline (vehicle) or the PAI-1 inhibitor for one week. Thereafter, the tumors were collected, and the M2 macrophages were stained with an anti-CD45 antibody, an anti-CD11b antibody, an anti-F4/80 antibody, and an anti-CD206 antibody, and the percentage of M2 macrophages in the tumor mass was analyzed with a flow cytometer.

Fig. 13 shows the results. Fig. 13(C) confirmed that the administration of the PAI-1 inhibitor reduced the number of macrophages in the tumor (bar graph on the left), and reduced the percentage of M2 macrophages, which are immunosuppressive cells, among macrophages (bar graph on the right). In other words, the administration of the PAI-1 inhibitor reduced intratumoral infiltration of immunosuppressive cells (M2 macrophages). The results of Experimental Example 11 and the results above indicate that the PAI-1 inhibitor suppresses PD-L1 expression in tumor cells (cancer cells), M2 macrophages, and other immunosuppressive cells to lead the inside of the tumor to an environment susceptible to immune attack.

### Experimental Example 14

1) The untreated group and 3) the single-administration group of PAI-1 inhibitor (cancer-bearing mice) of Experimental Example 11, after tumor engraftment, were orally administered with physiological saline (vehicle) or the PAI-1 inhibitor for one week. Thereafter, the tumors were collected, the cells were stained with an anti-CD45 antibody, an anti-CD4 antibody, an anti-CD25 antibody, and an anti-foxp3 antibody, and the percentage of regulatory T cells (Treg) in the tumor mass was analyzed with a flow cytometer.

Fig. 14 shows the results. Fig. 14(C) confirmed that the administration of the PAI-1 inhibitor reduced the percentage of regulatory T cells in the tumor. In other words, the administration of the PAI-1 inhibitor reduced the percentage of intratumoral infiltration of regulatory T cells. These results indicate that the PAI-1 inhibitor suppresses intratumoral infiltration of regulatory T cells, which are typical immunosuppressive cells that express PD-L1, and leads the inside of the tumor to an environment susceptible to immune attack.

### Experimental Example 15

1) The untreated group and 3) the single-administration group of PAI-1 inhibitor (cancer-bearing mice) of Experimental Example 11, after tumor engraftment, were orally administered with physiological saline (vehicle) or the PAI-1 inhibitor for one week. Thereafter, the tumor was collected, fixed with paraformaldehyde, and then embedded in paraffin to prepare tissue sections. The tissue sections were stained with an anti-SMAa antibody, and the percentage of cancer-associated fibroblasts (CAF) was observed under a microscope.

Fig. 15 shows the results. As shown in Fig. 15, the administration of the PAI-1 inhibitor reduced the percentage of intratumoral infiltration of cancer-related fibroblasts (densely stained cells, stained brown in the original drawing). These results indicate that the PAI-1 inhibitor suppresses intratumoral infiltration of cancer-related fibroblasts, which are typical immunosuppressive cells that express PD-L1, and leads the inside of the tumor to an environment susceptible to immune attack.

### Experimental Example 16

1) The untreated group and 3) the single-administration group of PAI-1 inhibitor (cancer-bearing mice) of Experimental Example 11, after tumor engraftment, were orally administered with physiological saline (vehicle) or the PAI-1 inhibitor for one week. Thereafter, the tumor was collected, the T cells were stained with an anti-CD45 antibody, an anti-CD3 antibody, an anti-CD4 antibody, an anti-CD8 antibody, and an anti-perforin antibody, and the percentage of cytotoxic T cells in the tumor mass was determined with a flow cytometer.

Fig. 16 shows the results. As shown in Fig. 16(A), the administration of the PAI-1 inhibitor increased the percentage of intratumoral infiltration of CD8⁺ T cells (effector T cells). Further, as shown in Fig. 16(B), the CD8⁺ T cells were cytotoxic T cells that express perforin. These results indicate that the administration of the PAI-1 inhibitor eliminated the immunosuppressive effect and increased the efficiency in tumor elimination by immunity.

### Experimental Example 17

Experimental Examples 1 and 2 and Experimental Examples 6 and 7 demonstrated that PAI-1 induces PD-L1 expression in cancer cells and in cell populations constituting the tumor-surrounding environment (immunosuppressive cells: TAMs, CAFs). In this Example, the following experiment was performed to confirm that PD-L1, whose expression is induced by PAI-1, suppresses cancer immunity.

As shown in Fig. 17(A), T cells isolated from the spleen of mice (C57BL/6) were stimulated overnight with an anti-CD3 antibody and an anti-CD28 antibody (activated T cells). At the same time, rPAI-1 was added to a culture medium of a cancer cell line (mouse malignant melanoma cells: B16F10), which was cultured overnight to induce PD-L1 expression. The T cells and the cancer cells (B16F10) collected from these were mixed and subjected to co-culturing overnight.

After culture, the cells were collected, stained with an anti-CD3 antibody, an anti-CD8 antibody, an anti-CD69 antibody, an anti-perforin antibody, and an anti-granzyme B antibody, and analyzed with a flow cytometer (Fig. 17(B)).

The degree of T-cell activation was analyzed using the expression level of CD69 antigen as an index. As shown in Fig. 17(C), the CD8⁺ T cells co-cultured with the cancer cells were activated, accompanied by an increase in CD69 expression; however, no increase in the CD69 expression was observed in the co-culture with cancer cells to which PAI-1 was added. Further, as shown in Figs. 17(D) and 17(E), the expression level of perforin and granzyme B showed the same tendency as CD69. Perforin and granzyme B are molecules released when CD8⁺ T cells damage cancer cells.

These results clarified that PD-L1 whose expression is induced by PAI-1 suppresses cancer immunity in cancer cells and in immunosuppressive cells.

### Experimental Example 18

In order to clarify the mechanism of induction of PD-L1 expression by PAI-1, rPAI-1 was added to a culture medium of a cancer cell line (B16F10); after culturing for 6 hours, the cells were collected, and the degree of phosphorylation of JAK/STAT molecules was analyzed.

Fig. 18 shows the results. Fig. 18(A) clarified that the addition of rPAI-1 increased the expression level of JAK1, TYK2, and STAT3, or induced phosphorylation. Further, Fig. 18(B) confirmed that the increase in the PD-L1 expression induced by rPAI-1 was suppressed by simultaneous addition of rPAI-1 and a STAT3 inhibitor (BP-1-102, C188-9). These results clarified that the induction of PD-L1 expression by PAI-1 is caused by the activation of the JAK/STAT pathway.

### Experimental Example 19

In order to clarify the mechanism of induction of PD-L1 expression by PAI-1, analysis was made in terms of LRP1, which is a receptor for PAI-1, and uPAR, which is a receptor for uPA. Specifically, an LRP1-deficient cancer cell line (B16F10; LRP1-KO) and a neutralizing antibody for inhibiting the binding between uPA and uPAR (uPAR blocking Ab) were prepared.

As shown in Fig. 19(A), induction of PD-L1 did not occur when rPAI-1 and the uPAR neutralizing antibody were added to a culture medium of a cancer cell line (B16F10) so as to induce PD-L1 expression. As shown in Fig. 19(B), induction of PD-L1 did not occur when rPAI-1 was added to a culture medium of the LRP1-deficient cancer cell line (LRP1-KO) so as to induce PD-L1 expression. These results clarified that PAI-1 forms a complex of PAI-1/LRP1/uPA/uPAR and transmits a signal for inducing PD-L1 expression.

## Claims

1. A programmed death ligand-1 (PD-L1) expression inhibitor comprising a compound having an inhibitory effect on plasminogen activator inhibitor-1 as an active ingredient.

2. The PD-L1 expression inhibitor according to claim 1, which is used as an immunostimulator.

3. The PD-L1 expression inhibitor according to claim 1, which is used as an immune checkpoint inhibitor for PD-L1.

4. The PD-L1 expression inhibitor according to claim 1, which is used as an inhibitor for exacerbation of tumor cells caused by PD-L1.

5. The PD-L1 expression inhibitor according to any one of claims 1 to 4, which is used as an enhancer of immunotherapy for tumors.

6. The PD-L1 expression inhibitor according to any one of claims 1 to 5, wherein the compound having a PAI-1 inhibitory effect is at least one member selected from the group consisting of a low-molecular-weight compound having a PAI-1 inhibitory effect, a PAI-1 neutralizing antibody and a fragment thereof, a peptide, an antisense oligonucleotide, siRNA, and an aptamer.

7. The PD-L1 expression inhibitor according to claim 6, wherein the low-molecular-weight compound having a PAI-1 inhibitory effect is a compound represented by Formula (I) below or a pharmaceutically acceptable salt, ester, or solvate thereof: 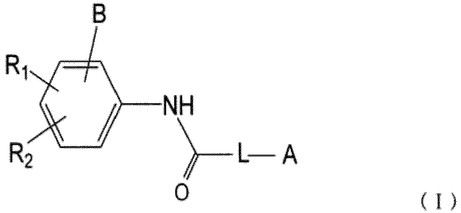 wherein
R₁ and R₂ are the same or different, and each represents hydrogen, halogen, substituted or unsubstituted C₁₋₆ alkyl, C₃₋₈ cycloalkyl, C₃₋₈-cycloalkyl-C₁₋₆-alkyl, C₃₋₈ cycloalkenyl, C₂₋₆ alkynyl, C₃₋₈-cycloalkyl-C₂₋₆-alkynyl, aryl, or 5- to 6-membered ring heteroaryl;
L is a single bond, - [(CH₂)_{M}-O-(CH₂)_{N}]_{Q}-CONH- (wherein M and N are the same or different, and each represents an integer of 1 to 6, and Q represents 0 or 1; -CONH- when Q is 0, and alkyleneoxyalkylene-CONH- when Q is 1), substituted or unsubstituted C₁₋₆ alkylene (some carbon atoms in the alkylene optionally form cycloalkyl), substituted or unsubstituted C₁₋₆ alkylene-O- (some carbon atoms in the alkylene optionally form cycloalkyl), substituted or unsubstituted C₁₋₆ alkylene-NHCO- (in the alkylene-NHCO-, some carbon atoms in the alkylene optionally form cycloalkyl), substituted or unsubstituted C₁₋₆ alkylene-NH-(in the alkylene-NH-, some carbon atoms in the alkylene optionally form cycloalkyl), substituted or unsubstituted C₂₋₆ alkenylene, substituted or unsubstituted C₂₋₆ alkynylene, -CO-, - NH-, 1,4-piperazidinyl, C₁₋₆ alkylene-1,4-piperazidinyl, or adamantylene;
A is a group represented by Formula (I-1) below: 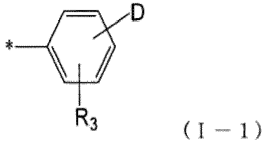 wherein
R₃ are the same or different, and each represents hydrogen, substituted or unsubstituted C₁₋₆ alkyl, or CF₃;
D is substituted or unsubstituted aryl, benzo-fused heteroaryl, or heteroaryl; substituted or unsubstituted C₃₋₈ cycloalkyl or C₃₋₈ heterocycloalkyl; substituted or unsubstituted C₃₋₈ cycloalkenyl or C₃₋₈ heterocycloalkenyl; substituted or unsubstituted C₁₋₆ alkyl; or adamanthyl; and
* is a binding site with L in Formula (I); and
B is COOR₄, wherein R₄ represents hydrogen or a group converted to hydrogen *in vivo.*

8. The PD-L1 expression inhibitor according to claim 7, wherein the compound represented by Formula (I) is a compound of Formulas (I) and (I-1), wherein
B is located at the ortho position of the benzene ring bound to imino,
L is a single bond, and
D is substituted or unsubstituted aryl or benzo-fused heteroaryl.

9. The PD-L1 expression inhibitor according to any one of claims 1 to 8, which is used in combination with another antitumor agent, another immunostimulator, and/or another immune checkpoint inhibitor.

10. A tumor chemotherapy agent comprising at least the PD-L1 expression inhibitor according to any one of claims 1 to 9 in combination with another antitumor agent, another immunostimulator, and/or another immune checkpoint inhibitor.

11. Use of a compound having a plasminogen activator inhibitor-1 inhibitory effect for producing a PD-L1 expression inhibitor, an immunostimulator, an immune checkpoint inhibitor, an inhibitor for exacerbation of tumor cells caused by PD-L1, or an enhancer of immunotherapy for tumors.

12. A compound having a plasminogen activator inhibitor-1 inhibitory effect, for use in at least one selected from the group consisting of the following (a) to (e):
(a) inhibiting PD-L1 expression in at least one cell selected from the group consisting of a tumor cell and an immunosuppressive cell;
(b) inhibiting an immune escape mechanism using PD-L1/PD-1 as an immune checkpoint;
(c) stimulating immunity, particularly stimulating immune depression caused by PD-L1;
(d) inhibiting exacerbation of tumor cells caused by PD-L1; and
(e) enhancing an immunotherapeutic effect on tumors.

13. A method for inhibiting PD-L1 expression in at least one cell selected from the group consisting of a tumor cell and an immunosuppressive cell in a patient, the method comprising administering an effective amount of a compound having a plasminogen activator inhibitor-1 inhibitory effect to the patient.

14. The method according to claim 13, wherein the patient is a patient expressing PD-L1 and having at least one cell selected from the group consisting of a tumor cell and an immunosuppressive cell.

15. The method according to claim 13 or 14, which is a method for inhibiting an immune escape mechanism using PD-L1/PD-1 as an immune checkpoint; a method for stimulating immunity of a tumor patient, particularly stimulating immune depression caused by PD-L1; a method for inhibiting exacerbation of tumor cells caused by PD-L1; or a method for enhancing an immunotherapeutic effect on tumors.
